# EUROPÄISCHE PATENTSCHRIFT

(11) **EP 1 003 711 B1**
(45) Veröffentlichungstag und Bekanntmachung des Hinweises auf die Patenterteilung: **07.11.2001**
(21) Anmeldenummer: 98946333.6
(22) Anmeldetag: 13.08.1998
(51) Int. Cl.: C07C 211/14, C07C 237/10, A61K 47/18

(54) **NEUE LIPOPOLYAMINE, DEREN DARSTELLUNG UND ANWENDUNG**
NOVEL LIPOPOLYAMINES, AND THE PREPARATION AND USE THEREOF
NOUVELLES LIPOPOLYAMINES, LEUR REPRESENTATION ET LEUR UTILISATION

(30) Priorität: 13.08.1997 DE 19735125; 31.07.1998 DE 19834683
(43) Veröffentlichungstag der Anmeldung: 31.05.2000
(73) Patentinhaber: Biontex Laboratories GmbH, 80807 München (DE)
(72) Erfinder: KLÖSEL, Roland, D-80933 München (DE); KÖNIG, Stephan, D-85567 Grafing bei München (DE)
(74) Vertreter: Forstmeyer, Dietmar, Dr. rer. nat., Dipl.-Chem.
(86) Internationale Anmeldenummer: EP9805156
(87) Internationale Veröffentlichungsnummer: WO9908997

(56) Entgegenhaltungen:
- EP-A- 0 394 111
- EP-A- 0 544 292
- WO-A-91/16024
- WO-A-94/05624
- WO-A-97/00241
- WO-A-97/03939

## Beschreibung

Positiv geladene Lipide (J. P. Behr, Bioconjugate Chem. 5, 382-389, 1994) werden in Form von Liposomen, Micellen oder als solche zur Einschleusung von biologisch aktiven Substanzen wie Peptiden, Peptoiden, Proteinen, PNA, antiviralen Wirkstoffen insbesondere aber DNA, RNA, Antisense-DNA/RNA oder Ribozymen in eukariotische Zellen (zum Beispiel Säuger-, Pflanzen-, oder Insektenzellen) eingesetzt. Lipopolyamine sind eine spezielle Klasse von kationischen Lipiden, die vergleichsweise hervorragende Transfektionseigenschaften zeigen. Unter Transfektion versteht man die Einschleusung von Erbmaterial in eukariotische Zellen.
Die Notwendigkeit DNA (zum Beispiel Plasmide, Cosmide, einzelsträngig oder doppelsträngig), RNA oder verwandte Stoffklassen, wie Antisense-DNA/RNA oder Ribozyme in eukariotische Zellen einzuführen, um beispielsweise erfolgreich Gentherapie betreiben zu können, führte zur Entwicklung einer Vielzahl von Transfektionsmethoden. Zur Einführung von Nukleinsäuren in eukariotische und insbesondere in Säugerzellen ist eine Vielzahl von Verfahren, wie zum Beispiel die CaPO₄-Präzipitationsmethode, die DEAE-Dextran-Methode, synthetische Polyamine (Polyethylenimin, Polylysin, PAMAM-Dendrimere), Methoden, welche die rezeptorvermittelte Endocytose nutzen, Elektroporation, Mikrobombardement, Mikroinjektion und Verfahren, die virale Capside als DNA-Carrier benutzen, bekannt. Eine weitere Methode wird Lipofektion (P. L. Felgner et al., Proc. Natl. Acad. Sci. USA 74, 7413 1987) genannt. Diese nützt die Tatsache, daß synthetische kationische Lipide in Form von Liposomen, Micellen oder als solche mit der negativ geladenen DNA Komplexe bilden. Stellt man die Mengenverhältnisse von DNA und kationischem Lipid so ein, daß die resultierenden Komplexe eine positive Nettoladung tragen, so besitzen sie eine hohe Affinität zu der negativ geladenen Membranoberfläche eukariotischer Zellen. Treffen solche DNA/Lipid-Komplexe auf Zellen, so kommt es zu einer Einschleusung des genetischen Materials in die Zelle. Der genaue Mechanismus, wie die DNA in die Zellen gelangt, ist noch weitgehend unbekannt, man vermutet jedoch, daß es entweder zu einer Fusionierung der kationischen Lipide mit der anionischen Zellmembran bei gleichzeitiger Ausschüttung der DNA in das Zellinnere kommt, oder daß die DNA/Lipid-Komplexe als ganzes über einen natürlichen Transportmechanismus der Zellen, die sogenannte Endozytose, in die Zelle gelangen und danach die DNA freigesetzt wird.
Liposomen sind in der Regel kugelartige Anordnungen von Lipiden in wäßrigen Lösungen mit "Bilayer-Struktur" und werden typischerweise in drei Klassifikationen eingeteilt (siehe N.Y. Academy Sciences Meeting: "Liposomes and their use in Biology and Medicine" von Dezember 1977): Multilamellare Vesikel (MLV, bis zu 10000 nm), kleine unilamellare Vesikel (SUV, 20-50 nm) und große unilamellare Vesikel (LUV, 600-30000 nm). Eine Reihe von Herstellungsmethoden für Liposomen ist bekannt und in "Liposome Technology" (Gregoriadis, CFC Press, N.Y. 1984) ,in "Liposomes" (Ostro, Marcel Dekker, N.Y. 1987) oder in Übersichtsartikeln von Lichtenberg et al. (Methods Biochem. Anal. 33, 337-462, 1988), Pagano und Weinstein (Ann. Rev. Biophysic. Bioeng. 7, 435-68, 1978), oder Szoka und Papahadjopoulos (Ann. Rev. Biophysic. Bioeng. 9, 467-508, 1980) beschrieben. Bekannte Methoden sind beispielsweise die "reverse-phase evaporation"-Methode und die Extrusionsmethode, bei der eine Lipidlösung durch eine mikroporöse Membran gepresst wird.
Liposomen werden typischerweise auch auf folgendem Weg hergestellt: Die Lipide werden in einem organischen Lösungsmittel aufgenommen. Durch Verdampfen des Lösungsmittels unter einem Strom von Stickstoff wird an der Glasgefäßwand ein dünner Lipidfilm erzeugt. Zugabe von Wasser oder wässriger Pufferlösung hydratisiert diesen Film. Die erhaltene Lösung wird zuletzt mit Ultraschall behandelt.
Kationische Lipide gewinnen zunehmende Bedeutung in der Gentherapie. Dabei werden in vivo mit verschiedenen Verfahren Körperzellen transfektiert, indem Komplexe aus Carrier und DNA intradermal, intramuskulär, intraperitoneal, intravenös, subkutan, intranasal, in Liquorräume oder direkt in Tumore verabreicht werden oder Körperzellen entnommen, transfektiert und wieder reimplantiert werden. Eine in diesem Zusammenhang favorisierte Methode war bis vor einiger Zeit die Einbringung des genetischen Materials durch virale Carrier. Diese Methode besitzt jedoch die Gefahr der Rückmutation zu einem pathogenen Virus. Desweiteren wird die eingeschleuste DNA stabil in das Erbgut eingebaut, so daß eine Steuerung der Therapie oder eine Rückführung der Zellen in ihren ursprünglichen Zustand nicht mehr möglich ist. Zudem besitzen virale Carrier Restriktionen bezüglich der Größe der einzuschleusenden DNA. Modifizierte DNA oder RNA wird durch Viren nicht übertragen. Außerdem können nur sich teilende Zellen auf diesem Wege transfektiert werden.
Weitere Gefahren, die bei der Anwendung von viralen Carriersystemen zu berücksichtigen sind, sind die mögliche Aktivierung von Oncogenen und eine Immunreaktion des behandelten Organismus.
Die Transfektion mit kationischen Lipiden ist diesen Restriktionen hingegen nicht unterworfen. Die Transfektion verläuft in der Regel transient, das heißt, die transfektierte DNA oder RNA wird nur für eine bestimmte Zeit exprimiert, da sie nicht in das Erbgut eingebaut wird und durch Nukleasen mit der Zeit abgebaut wird. Auf diese Weise kann Gentherapie dosiert und reversibel gemacht werden. Restriktionen bezüglich der Größe der DNA bestehen nicht und auch modifizierte DNA oder RNA (z.B. Antiseuse-DNA/RNA oder durch Einbau modifizierter Nukleotide stabilisierte Ribozyme) kann mittels kationischer Lipide in Zellen eingeschleust werden. Auch sich nicht teilende Zellen, wie zum Beispiel Nervenzellen, können durch kationische Lipide transfektiert werden. Desweiteren wurde bisher kein immunogenes Verhalten von kationischen Lipiden bei in vivo Versuchen gefunden.
Während die in vivo-Anwendung von Mikroinjektion und Elektroporation aus Verfahrensgründen nicht möglich erscheint, besitzen die CaPO₄- und DEAE-Dextran-Methoden verglichen mit der Lipofektion eine schlechtere Transfektionseffizienz.
Unter den kationischen Lipiden gibt es eine Klasse, die die bekannt hohe Affinität zwischen Polyaminen (z.B. Spermin, Spermidin) und DNA zur Transfektion ausnützen, sogenannte Lipopolyamine. Die Polyamine sind dabei linear oder verzweigt aufgebaut und enthalten Ethylen; Propylen-; oder Butylengruppen zwischen den Aminofunktionen. Die Polyamine sind auf unterschiedlichste Weise mit einem lipophilen Rest verbunden.
Beispielsweise wird das bei physiologischem pH-Wert positiv geladene Spermin mit einem hydrophilen Rest, in manchen Fällen über einen Spacer, verknüpft. Spermin bildet stabile Komplexe mit DNA und ähnlichen Verbindungen, indem es über Wasserstoffbrückenbindung in der Furche der DNA gebunden wird.
Die ersten solchen Liposperminderivate wurden von Behr, J. P.et al. (Proc. Natl. Acad. Sci. USA 86: 6982-6986; 1989; EP 0394111) synthetisiert. Dabei verknüpften sie Carboxyspermin über einen Spacer mit zwei unterschiedlichen hydrophilen Resten. Die Struktur des dabei erhaltenen 5-Carboxyspermylglycindioctadecylamid (DOGS) ist: 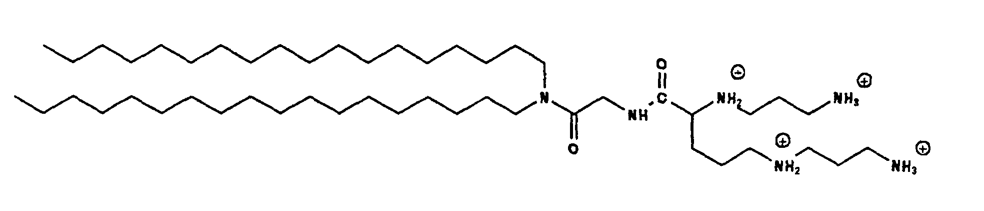 DOGS ist als Transfectam™ (Promega) kommerziell erhältlich.
Die zweite von Behr et al. entwickelte Verbindung ist Dipalmitoylphosphatidylethanolamin-5-carboxyspermylamid (DPPES):

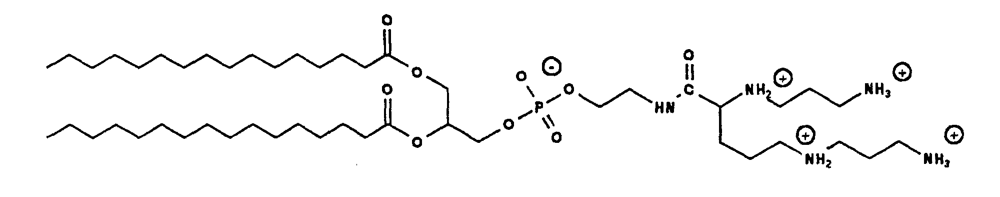

Ein weiteres Liposperminderivat wird von P. L. Felgner et al. in WO 9116024 unter der Bezeichnung L-Spermin-5-carboxyl-3-(DL-1,2-dioleoyldimethylaminopropyl-β-hydroxyethylamin) beansprucht. In der Patentschrift beschrieben ist jedoch L-Spermin-5-carboxyl-3-(DL-1,2-dipalmitoyl-dimethylaminopropyl-β-hydroxyethylamin):

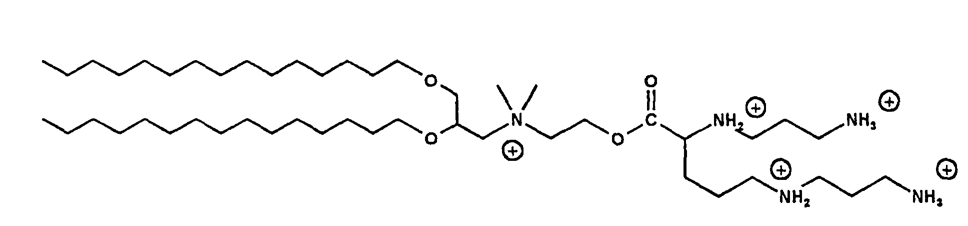

Gebeyehu, G. et al. beschreiben in WO 9405624 die Verbindung N-[N-(5-Carboxyspermyl)aminoethyl] N, N-dimethyl-2,3-bis (9-octadecenyloxy) 1-propanammomum tetra (trifluoracetat), welches kommerziell als Lipofectamin™ (Gibco-BRL : Life Technologies Inc.) erhältlich ist.

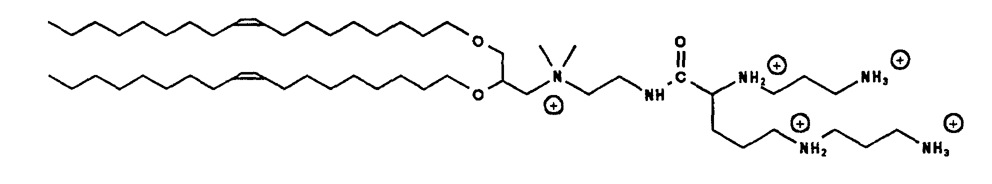

Von Der Eltz et al. beschreiben in WO 9700241 die Verbindung 2-( 6-Carboxy-spermyl)-1,3-dioleoyloxy-propylamid, die unter dem Namen DOSPER auf den Markt gebracht wurde (Boehringer Mannheim GmbH).

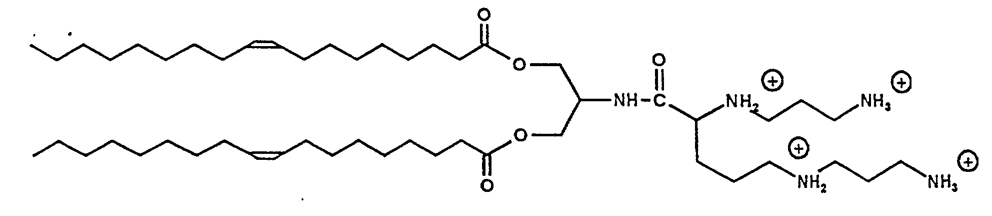

Die bisher genannten Verbindungen wurden alle über eine Amid- oder Esterverknüpfung eines linearen Polyamins, welches eine Carboxyfunktion als Seitenkette trägt, i.e. von Carboxyspermin zu einem lipohilen Rest hergestellt. Spätere Veröffentlichungen zeigen schließlich auch andere Verknüpfungsweisen von linearen Polyaminen zu den lipophilen Resten, z.B durch eine Amid- oder Carbamatverknüpfung zu einer endständigen Aminofunktion (lineare Struktur) oder inneren Aminofunktion ("T-shape-structure") des linearen Polyamins. Beispiele finden sich in den Veröffentlichungen DE 1963189, WO 9640726, WO 9640725, WO 9618372, WO 9746223,WO 9802190 und WO 9802191.
Auch verzweigte Polyamine wurden beschrieben (G. Byk et al, J. Med. Chem, 41, 224-235, 1998).

In der Druckschrift WO-A-97/03939 werden kationische Lipide auf Amidbasis offenbart, die in Lipidaggregaten zur Einführung von Makromolekülen in Zellen geeignet sind.

Trotz großer Fortschritte auf dem Gebiet der Transfektion mittels kationischer Lipide, verbleibt ein Bedarf an einer größeren Auswahl solcher Lipide. Der Grund dafür liegt darin, daß bis heute kein kationisches Lipid gefunden wurde, daß mit allen Zelltypen befriedigende Ergebnisse liefert. Da verschiedene Zelltypen "sich in ihrer Membranzusammensetzung unterscheiden, ist es nicht verwunderlich, daß verschiedene Kompositionen von Lipiden und verschiedenartige Lipidtypen für eine effektive Transfektion unterschiedlicher Zellen benötigt werden.
Da über den eigentlichen Transfektionsschritt bis heute noch wenig bekannt ist, ist die Entwicklung von neuen kationischen Lipiden weitgehend empirisch. Wichtige zu beachtende Gesichtspunkte für das Design solcher Lipide sollten daher ihre physikalischen Eigenschaften bezüglich der Ausbildung von Liposomen oder Micellen, die Eigenschaften der gebildeten Liposomen oder Micellen, die Toxizität gegenüber den zu transfektierenden Zielzellen, desweiteren die Stabilität der Lipide, deren Metabolisierbarkeit und die Möglichkeit einer in vivo-Anwendung sein.
Die erfindungsgemäß zu lösende Aufgabe bestand daher darin, neue kationische Lipide mit hoher Wirksamkeit und möglichst breitem Wirkungsspektrum in Kombination mit guter Stabilität und geringer Toxizität zu finden. Bei der vorliegenden Erfindung wurde nun überraschenderweise gefunden, daß die Kombination von Polyaminen als Kopfgruppe mit einem speziellen lipohilen Rest zu besonders guten Eigenschaften in Hinblick auf Effizienz und Stabilität führt:

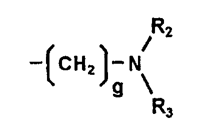

Die Reste R sind dabei gesättigte oder ungesättigte lineare oder verzweigte Alkylketten, vorzugsweise mit 10 bis 20 Kohlenstoffatomen.
Als Erklärung mögen folgende Hypothesen dienen. Von besonderer Bedeutung für eine erfolgreiche Transfektion hat sich die Bildung eines sogenannten DNA-Lipid-Komplexes erwiesen. Beim Zusammentreffen von DNA und kationischen Lipiden, die als Liposomen- oder Micellenformulierungen eingesetzt werden, kommt es zu einer spontanen Kondensation der DNA unter Zusammenbruch der liposomalen oder micellenartigen Organisationsstruktur der kationischen Lipide. Es bildet sich ein DNA-Lipid-Komplex aus, in dem die DNA in einem multilamellaren Komplex mit "Bilayerstruktur" aus den besagten Lipiden eingebettet ist (J. O. Rädler et al. Science, 275, 810, 1997). Ist z.B. die Spermingruppe als DNA-affine Kopfgruppe erst einmal an die DNA gebunden, kommt somit der Flexibilität und Symmetrie des restlichen Molekülteils (lipophiler Rest) besondere Bedeutung zu, da die Alkylgruppen in der Lage sein müssen, sich möglichst parallel auszurichten, um über Van-der-Waals-Kräfte stabile "Bilayer-Strukturen" ausbilden zu können.
Die bisher vorgeschlagenen Verbindungen sind in der Regel wenig symmetrisch aufgebaut, was eine parallele Ausrichtung der Alkylreste erschwert, und beinhalten allesamt Ester, Ether oder Amidverknüpfungen zwischen den Alkylresten und einem Grundgerüst bzw. einem Spacer. Diese Verbindungen sind daher in ihrer Flexibilität aufgrund der festgelegten Bindungswinkel und im Falle von Amidverknüpfungen (z.B. bei Verbindungen aus G. Byk et al, J. Med. Chem., 41, 224-235, 1998, EP 0394111, WO 9618372, WO 9746223,WO 9802190 und WO 9802191) aufgrund vorhandener Resonanzstrukturen stark eingeschränkt.

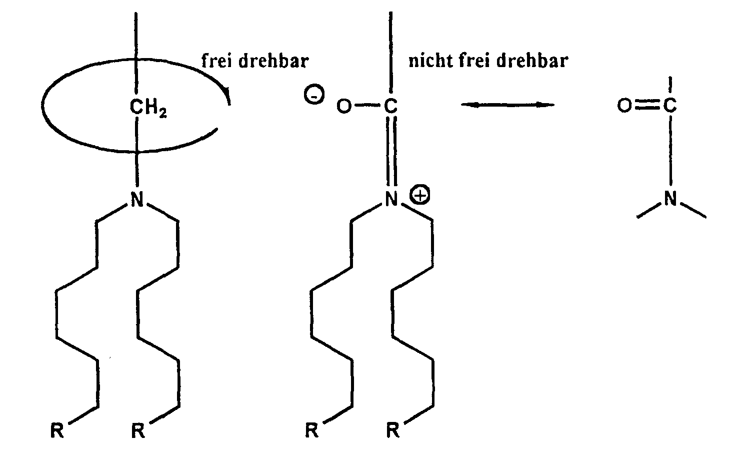

In der Resonanzstruktur fiir Amide trägt das O-Atom eine negative und das Amid-N-Atom eine positive Ladung. Es resultiert eine planare Anordnung der an der Amidbindung beteiligten Atome. Eine freie Rotation der Amidbindung zwischen Carbonyl.Kohlenstoff und dem Stickstoffatom ist nicht möglich. Diese Eigenschaft wirkt sich beispielsweise in ganz besonderer Weise auf die Struktur von Proteinen aus. (Sekundär-, Tertiär- und Quartärstruktur), deren Peptidbindungen nichts anderes als Amidverknüfungen sind. Erst die starren, nichtflexiblen Amidverknüpfungen geben dem Protein seine biologisch notwendige Struktur.
Die erfindungsgemäßen Verbindungen (siehe auch unten) verknüpfen die Alkylreste R₂ und R3 über ein tertiäres Stickstoffatom (Amin). Damit ist eine hohe Symmetrie und durch die Möglichkeit des "Durchschwingens" des freien Elektronenpaares (Pseudorotation) am Stickstoffatom maximale Flexibilität gewährleistet. Somit besteht bei diesen Verbindungen die optimale Voraussetzung zur parallelen Ausrichtung der Alkylreste (trotz an der DNA fest verankerter Kopfgruppen) und somit zur Bildung stabiler DNA-Lipid-Komplexe. Die Stabilität wird insbesondere auch durch eine große Überlappung der van der Waals-Regionen der Alkylgruppen bewirkt.
Eine weitere Erklärungsmöglichkeit für die besonders guten Eigenschaften der beanspruchten Verbindungen besteht darin, daß diese Verbindungen aufgrund des nicht an der DNA-Komplexbildung beteiligten basischen Stickstoffatoms, welches die Alkylreste trägt, Pufferungsvermögen im physiologischen pH-Bereich besitzen. Diese Eigenschaft fördert auf dem Weg der Endozytose die osmotischen Zerstörung der Endosomen, so daß die DNA um so besser in das Zytosol freigesetzt wird (J. P. Behr, Gene Therapy, 3,1010-1017, 1996).

Es hat sich insbesondere gezeigt, daß erfindungsgemäßge Verbindungen, bei denen die Polyamine der Kopfgruppe, die an den lipophilen Rest R₁ gebunden ist, mindestens drei Stickstoffatome aufweisen, besonders gute Eigenschaften aufweisen; derartige Verbindungen werden daher besonders bevorzugt.

Die vorliegende Erfindung betrifft somit neue Lipopolyamine der allgemeinen Formel I, die in der Lage sind, biologisch aktive Moleküle in eukariotische Zellen zu transportieren: 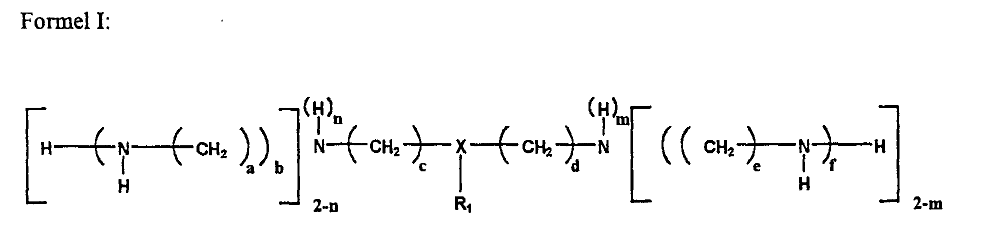 die bei Vorhandensein eines Asymmetriezentrums in der D-,L- oder DL-Form vorliegen, einschließlich ihrer Salze, wobei
R₁ ein lipophiler Rest folgender allgemeinen Formel ist: 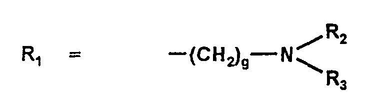 in welcher R₂ und R₃ unabhängig voneinander Dodecyl, Dodecenyl, Tetradecyl, Tetradecenyl, Hexadecyl, Hexadecenyl, Octadecyl, Octadecenyl oder andere Alkylreste, die in allen möglichen Kombinationen gesättigt, ungesättigt, verzweigt, unverzweigt, fluoriert oder nicht fluoriert sein können, und aus 5, 6, 7, 8, 9, 10, 11, 12, 13, 14, 15, 16, 17, 18, 19, 20, 21, 22, 23, 24, 25, 26, 27, 28, 29 oder 30 Kohlenstoffatomen aufgebaut sind,
und X eine der folgenden Gruppierungen ist: 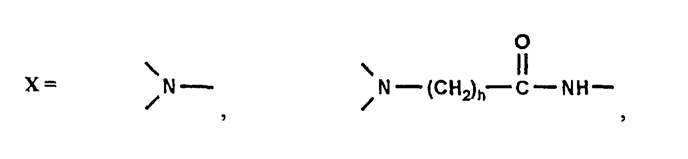 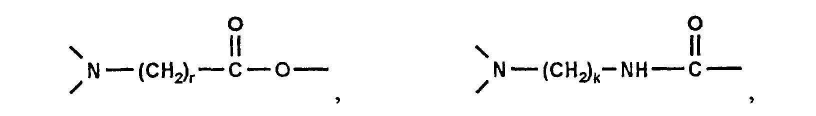 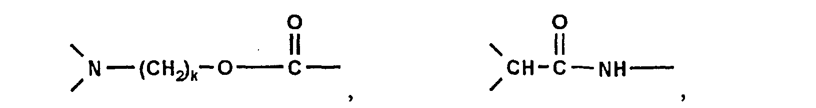 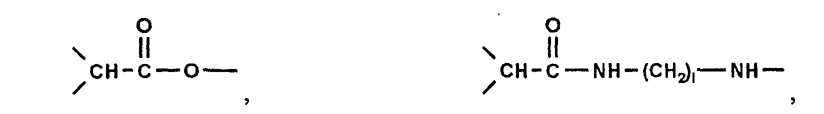 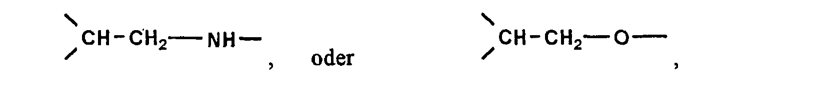 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können,
   oder
   wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können,
   oder
   wobei m = 0 und n = 2 sind and für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können,
   oder
   wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können,
   oder
   wobei m = 1 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können,
   oder
   wobei m = 2 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, h = 0,1,2,3,4,5 oder 6, r = 0,1,2,3,4,5 oder 6, k = 0,1,2,3,4,5 oder 6 und 1 = 0,1,2,3,4,5 oder 6 sein können, wobei vorzugsweise maximal 50%, besonders bevorzugt 30%, der H-atome durch F-Atome ersetzt sind.

Vorzugsweise ist g > 1. Bei bevorzugten Verbindungen ist c=3 und/oder d=3. Bei besonders bevorzugten Verbindungen ist a=0 oder 3 und/oder e=0 oder 3 und/oder b=0 oder 1 und/oder f=0 oder 1.

Von besonderem Interesse sind dabei Verbindungen und ihre Salze mit folgenden Strukturen: 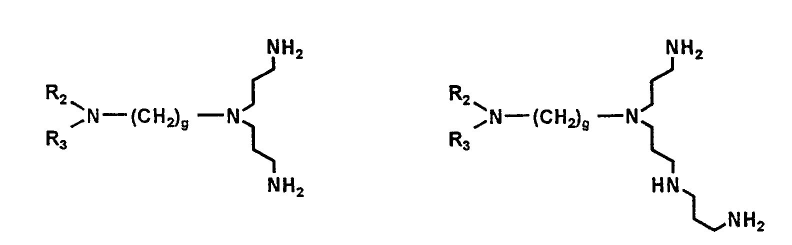 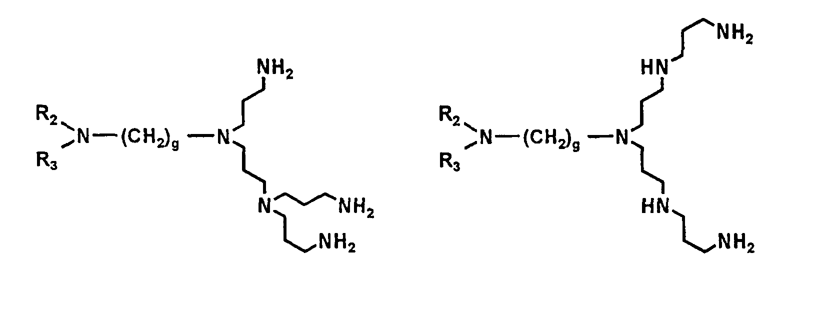 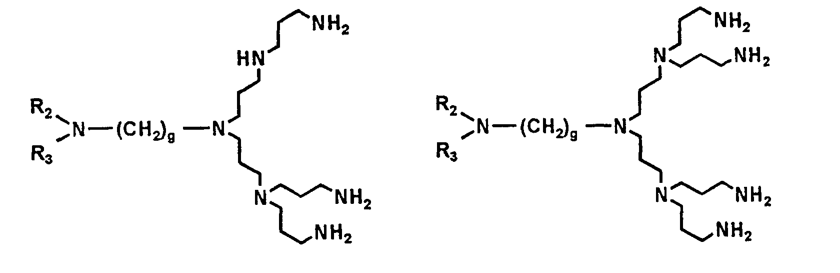 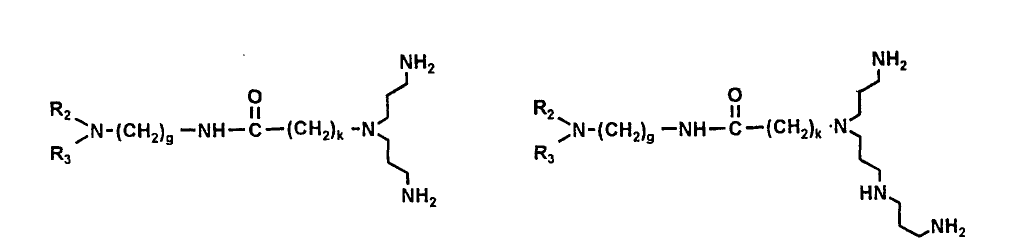 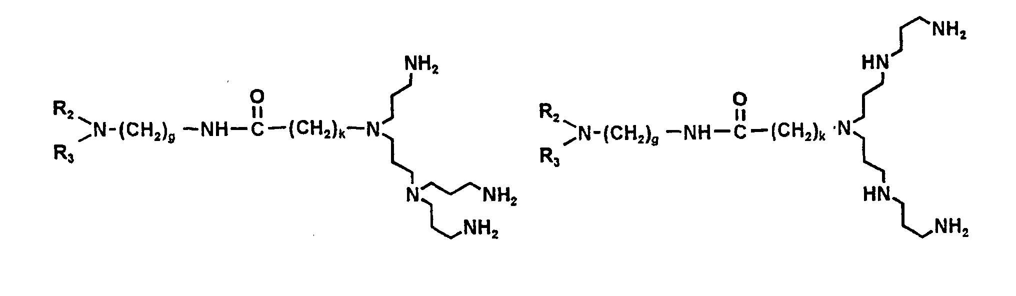 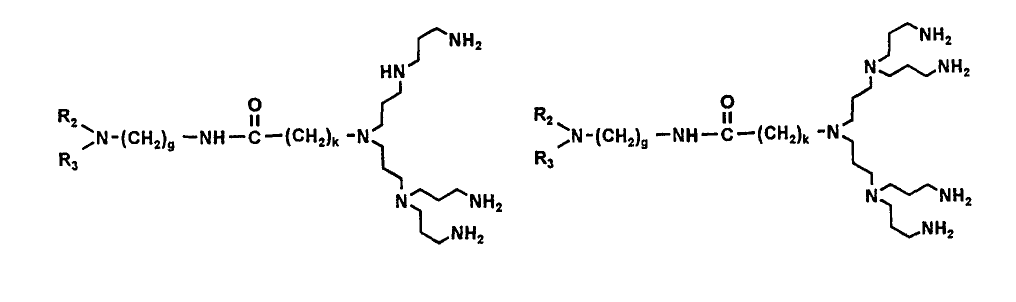 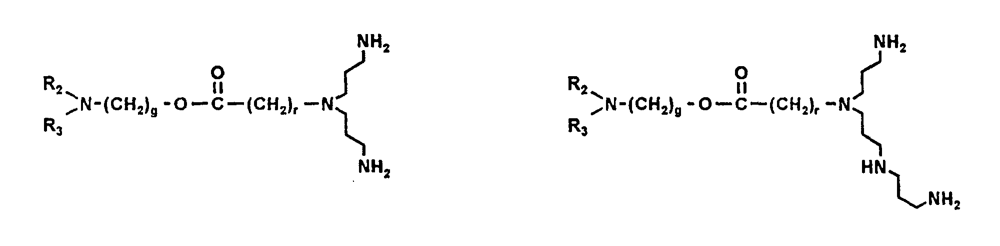 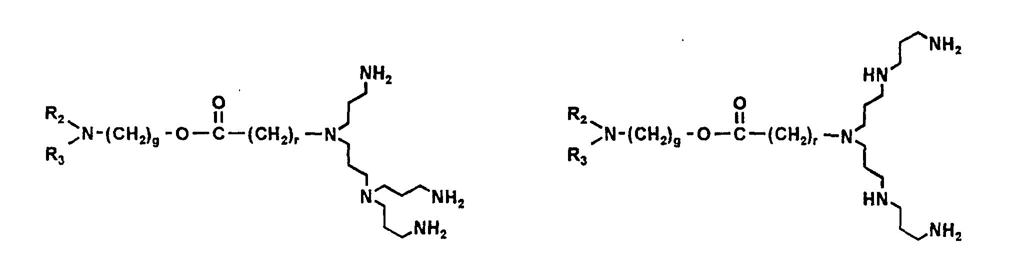 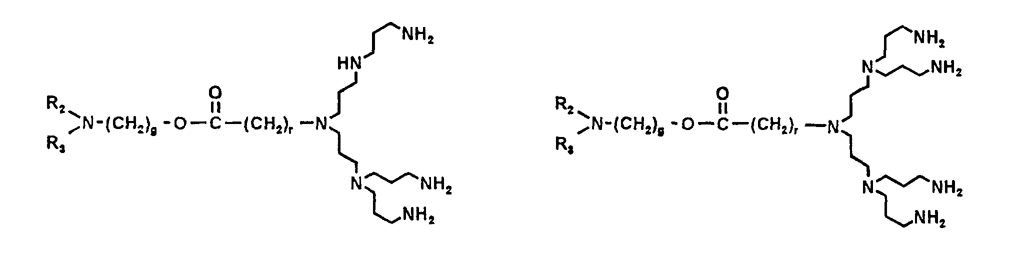 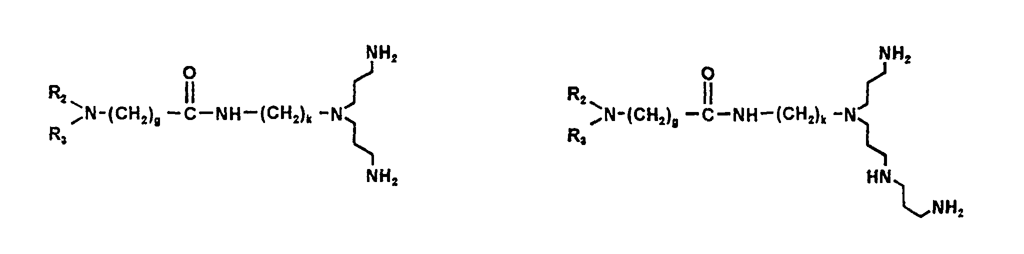 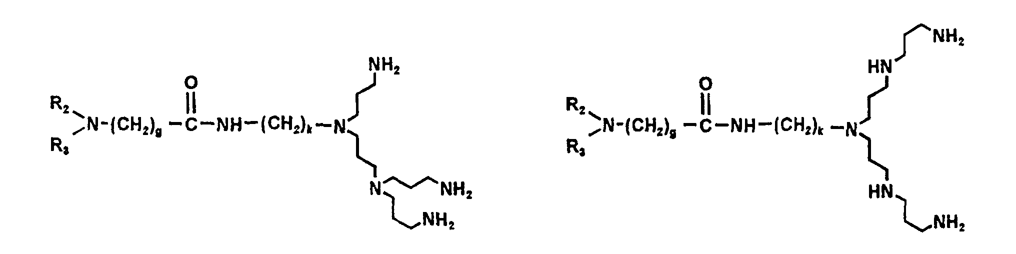 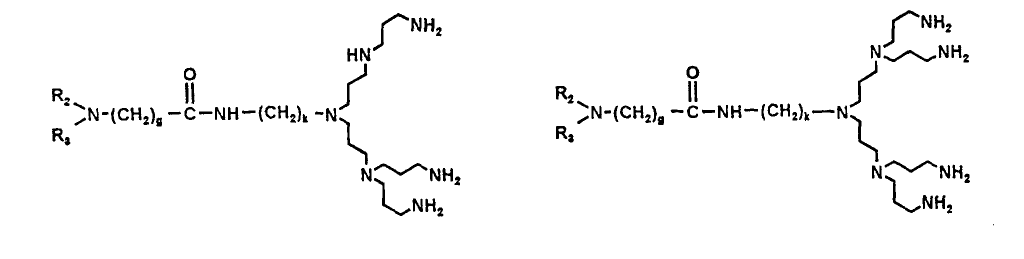 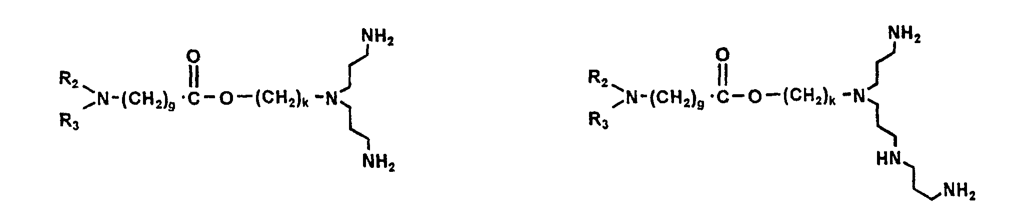 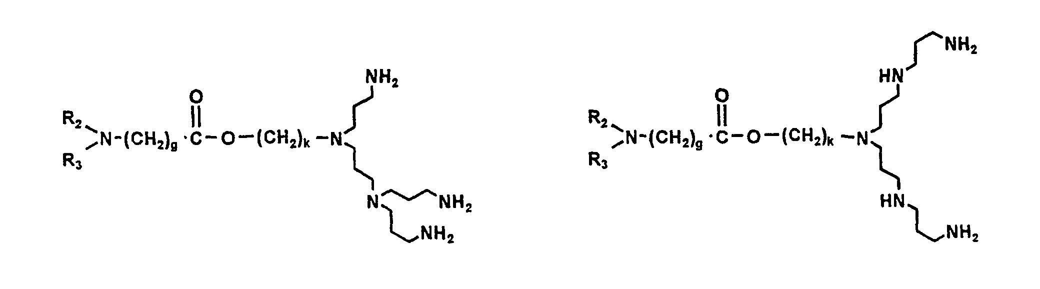 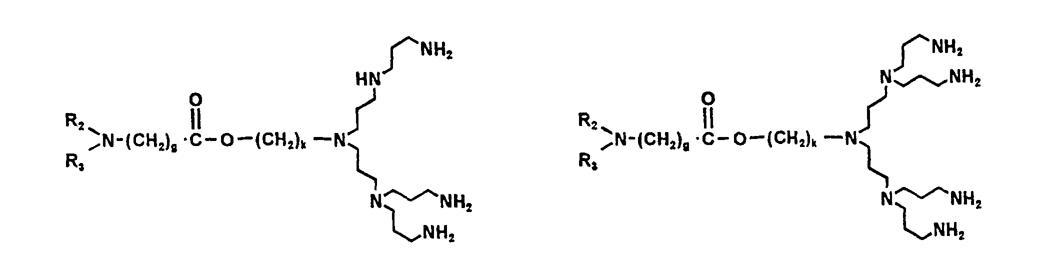 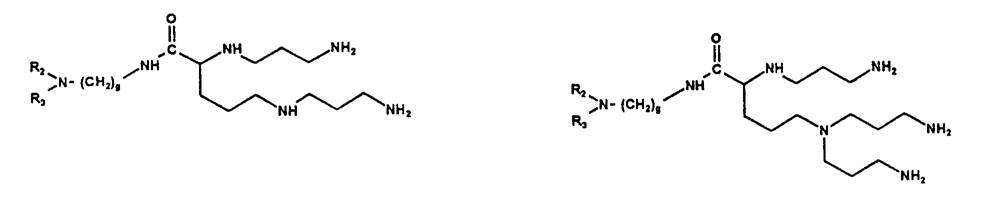 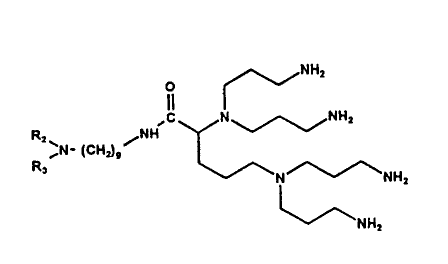 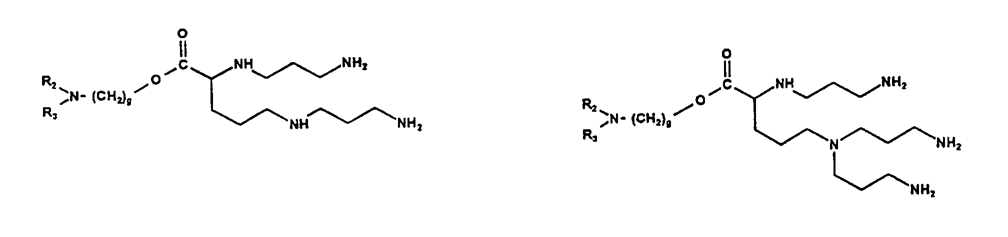 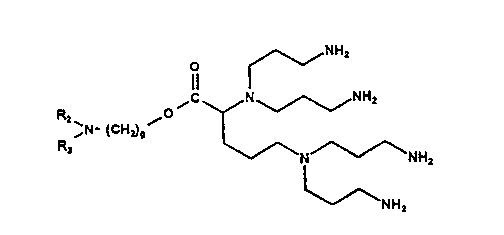 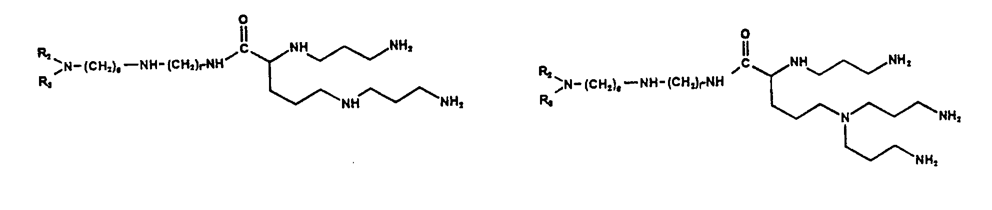 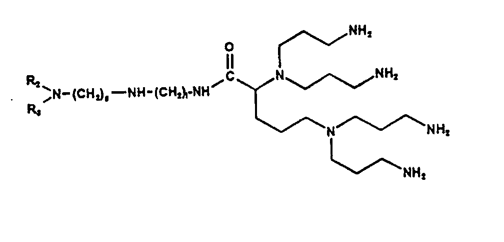 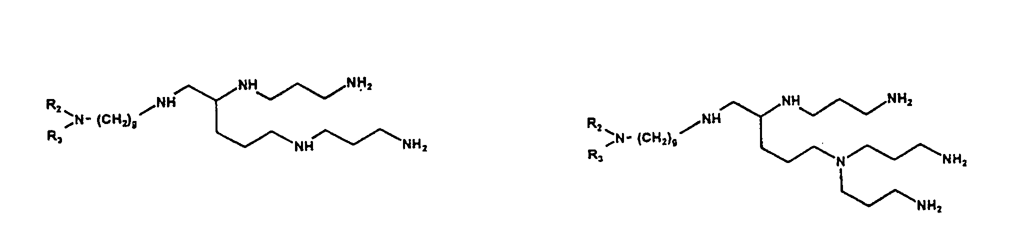 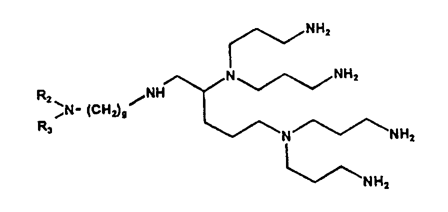 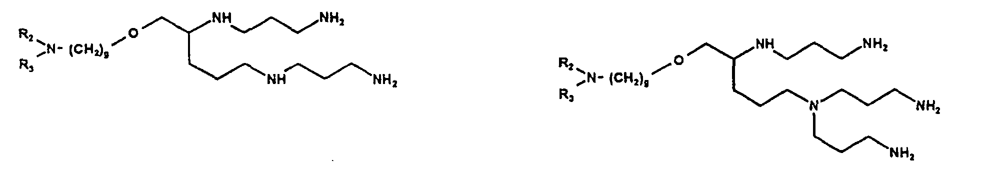 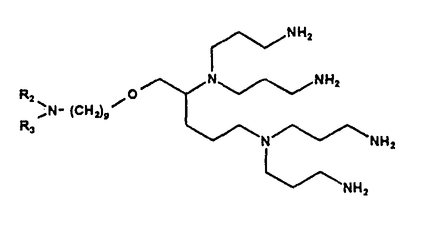 wobei h = 0,1,2,3,4,5 oder 6, r = 0,1,2,3,4,5 oder 6, k = 0,1,2,3,4,5 oder 6, 1 = 0,1,2,3,4,5 oder 6 and wobei g = 1,2,3,4,5,6,7 oder 8 sein können, R₂ und R₃ unabhängig voneinander Dodecyl, Dodecenyl, Tetradecyl, Tetradecenyl, Hexadecyl, Hexadecenyl, Octadecyl oder Octadecenyl sein können.

Von ganz besonderem Interesse ist dabei folgende Verbindung und ihre Salze:

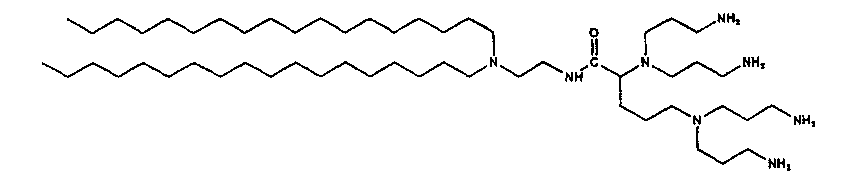

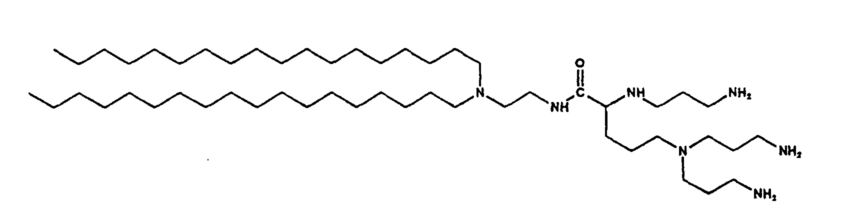

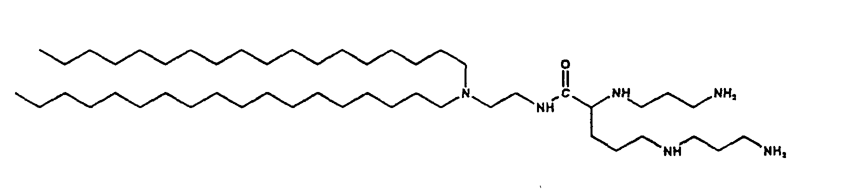

Von weiterem besonderen Interesse sind Zusammensetzungen oder Formulierungen (die Liposomen oder Micellen enthalten), die mindestens eine der erfindungsgemäßen Verbindungen mit oder ohne Colipide, wie z.B. Dioleoylphosphatidylethanolamin (DOPE), Dioleoylphosphatidylcholin Cholesterol oder Cholesterylamin enthalten. Die Zusammensetzungen oder Formulierungen (die Liposomen oder Micellen enthalten) können übliche Zusatzstoffe, Träger, Adjuvantien etc. enthalten.

Von weiterem besonderen Interesse sind Verfahren zum Einbringen von biologisch wirksamen Verbindungen, wie DNA, RNA, Ribozymen, Antisense-DNA, PNA, Peptiden, Peptoiden und Proteinen in eukariotische Zellen, wobei die erfindungsgemäßen Verbindungen, Zusammensetzungen oder Formulierungen mit den einzubringenden (biologisch aktiven) Verbindungen komplexiert und die entstandenen Komplexe mit eukariotischen Zellen in vivo oder in vitro in Kontakt gebracht werden.

Von weiterem besonderen Interesse ist die Verwendung der erfindungsgemäßen Verbindungen, Zusammensetzungen oder Formulierungen (zur Herstellung eines Medikaments oder eines Reagenzes) zum Einbringen von biologisch wirksamen Verbindungen, wie DNA, RNA, Ribozymen, Antisense-DNA, PNA,Peptiden, Peptoiden und Proteinen in eukariotische Zellen in vivo oder in vitro.

Von weiterem besonderen Interesse ist die Verwendung der erfindungsgemäßen Verbindungen, Zusammensetzungen oder Formulierungen (zur Herstellung eines Medikaments oder eines Reagenzes) zum Einbringen von biologisch wirksamen Verbindungen, wie DNA, RNA, Ribozymen, Antisense-DNA, PNA, Peptiden, Peptoiden und Proteinen in eukariotische Zellen in vivo oder in vitro in Kombination mit sogenannten "Enhancern", die die Wirkung der erfindungsgemäßen Verbindungen verstärken.

Die erfindungsgemäßen Lipide enthalten also Polyamine als DNA-affine Kopfgruppen, die gegebenenfalls über einen Spacer an einen speziellen lipophilen Rest gebunden sind.
Ein besonderer Wert der erfindungsgemäßen Lipide liegt in ihrer Stabilität in Lösung, bei gleichzeitiger geringer Toxizität für die Zelle in Kombination mit außergewöhnlich guten Transfektionseigenschaften.
So konnte gezeigt werden, daß die erfindungsgemäßen Verbindungen, im Vergleich mit Lipofectamin™ (Gribco-BRL : Life Technologies Inc.), dem bis dato in der Fachwelt anerkannt wirksamsten Transfektionsreagenz aus der Gruppe der Lipopolyamine, bessere Transfektionseigenschaften besitzen, wenn sie die gleiche Kopfgruppe tragen. Die Transfektionseffizienz ist dabei nicht nur jeweils im serumfreien und serumhaltigen Milieu höher, sondern es zeigt sich auch, daß ein hohes Niveau der Transfektionseffizienz über ein wesentlich breiteres DNA-Lipid-Verhältnis erreicht werden kann.

Die erfindungsgemäßen Verbindungen können dadurch hergestellt werden, daß eine Aminogruppe eines Diamins gegebenenfalls in Gegenwart einer Base mit einem Alkylierungsreagenz umgesetzt und die andere Aminogruppe mit einem gegebenenfalls BOC-geschützten Carboxypolyalkylamin gekuppelt wird. Dabei ist die andere Aminogruppe vorzugsweise im ersten Reaktionsschritt z.B. mit einer BOC-Gruppe geschützt, die vor Umsetzung mit dem Carboaypolyalkylamin entfernt wird. Als Alkylierungsreagenz kann ein Alkylbromid wie Stearylbromid eingesetzt und als Base z.B. Triethylamin oder 4-Methylmorpholin eingesetzt werden.

Nachstehend wird ein entsprechender Syntheseweg am Beispiel einer der ganz besonders interessanten Verbindungen, nämlich von N-[2,5-Bis [(3-aminopropyl) amino]-1-oxopentyl]-N',N'-dioctadecylethylendiamin, erläutert.

Dabei wird von Alkylendiamin ausgegangen, bei dem eine Aminogruppe mit N-tert.-Butyloxycarbonyl geschützt (BOC geschützt) ist. Die freie Aminogruppe wird durch Alkylbromide wie z.B. Stearylbromid in Anwesenheit einer Base derivatisiert. Anschließend wird die geschützte Aminofunktion in an sich üblicher Weise deblockiert und in an sich üblicher Weise an an allen (reaktiven) Aminofunktionen BOC-geschütztes Carboxypolyalkylamin (hier BOC-geschütztes Carboxyspermin) gekuppelt. Nach in an sich üblicher Weise erfolgendem Deblockieren dieser Kopfgruppe erhält man die gewünschten Verbindungen, je nach Aufarbeitung als freies Amin oder in Form ihrer Salze.

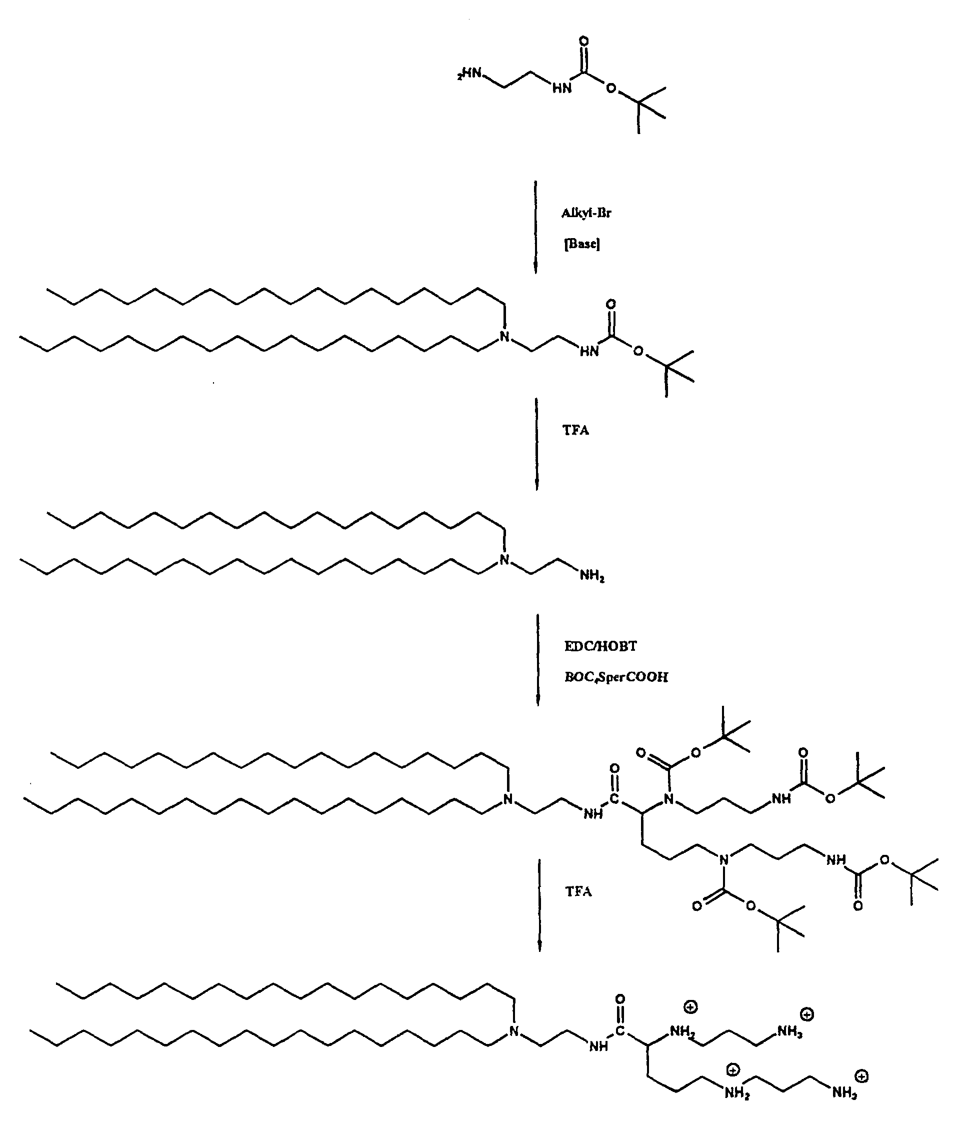

In einer weiteren Ausführungsform wird ein gegebenfalls an einer Aminogruppe geschütztes Diamin mit Acrylnitril umgesetzt und das Produkt mit einem Alkylierungsreagenz, wie Stearylbromid, gegebenenfalls in Gegenwart einer Base umgesetzt. Das Produkt kann dann hydriert werden.

So kann eine weitere bevorzugte Verbindung, nämlich N,N-Bis-(3-aminopropyl)-N',N'-dioctadecylethylendiamin hergestellt werden. Diese Verbindung wird ausgehend von N-tert.-Butyloxycarbonylethylendiamin (BOC-Ethylendiamin) durch Umsetzen mit Acrylnitril, Alkylierung mit Alkylbromid und anschließender Hydrierung gewonnen. Die anschließenden Schritte dienen der Aufreinigung.

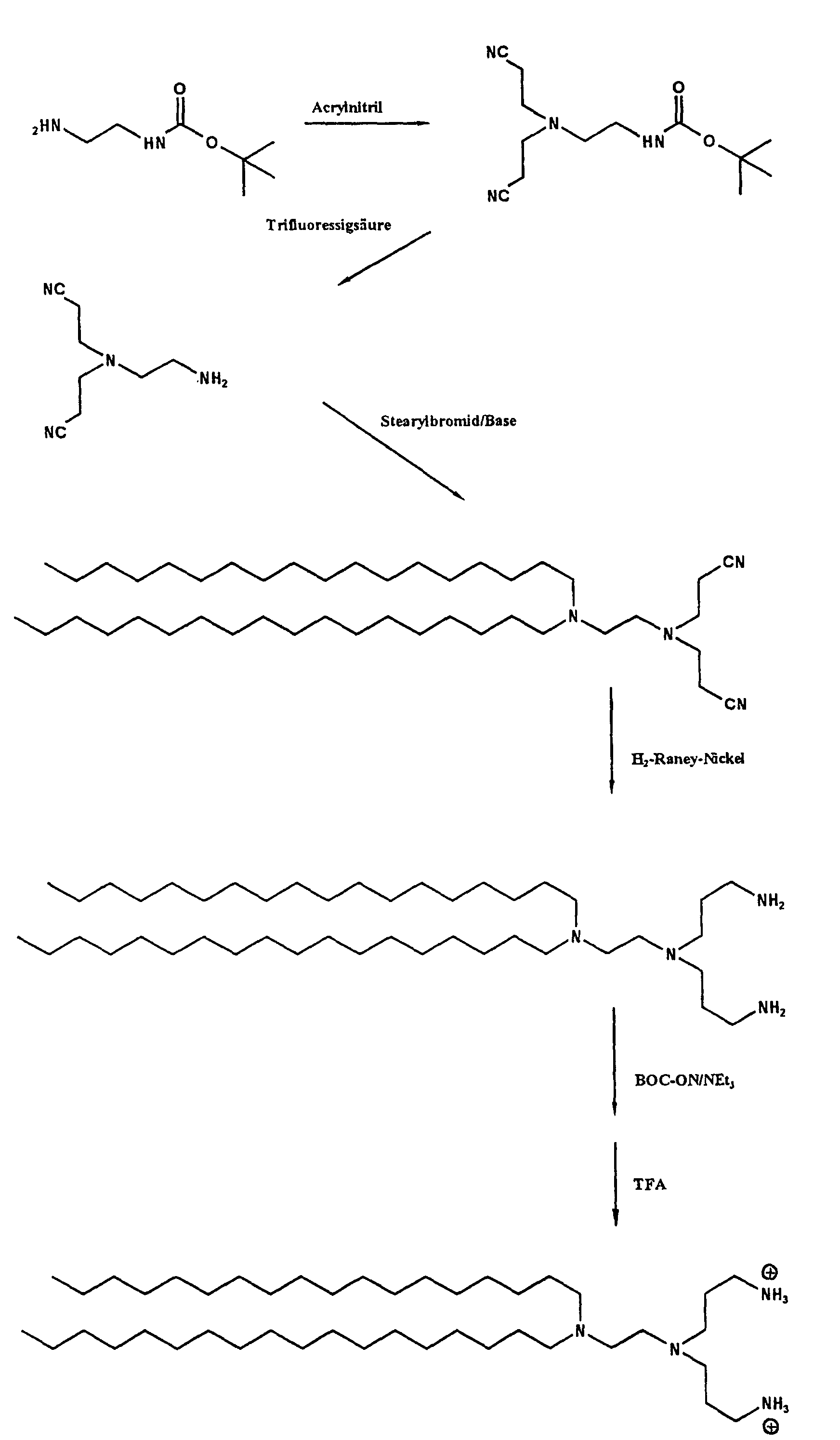

Ein alternativer Weg zum gleichen Molekül ist die Alkylierung von N,N-Dioctadecylethylendiamin mit N-BOC-3-brompropylamin:

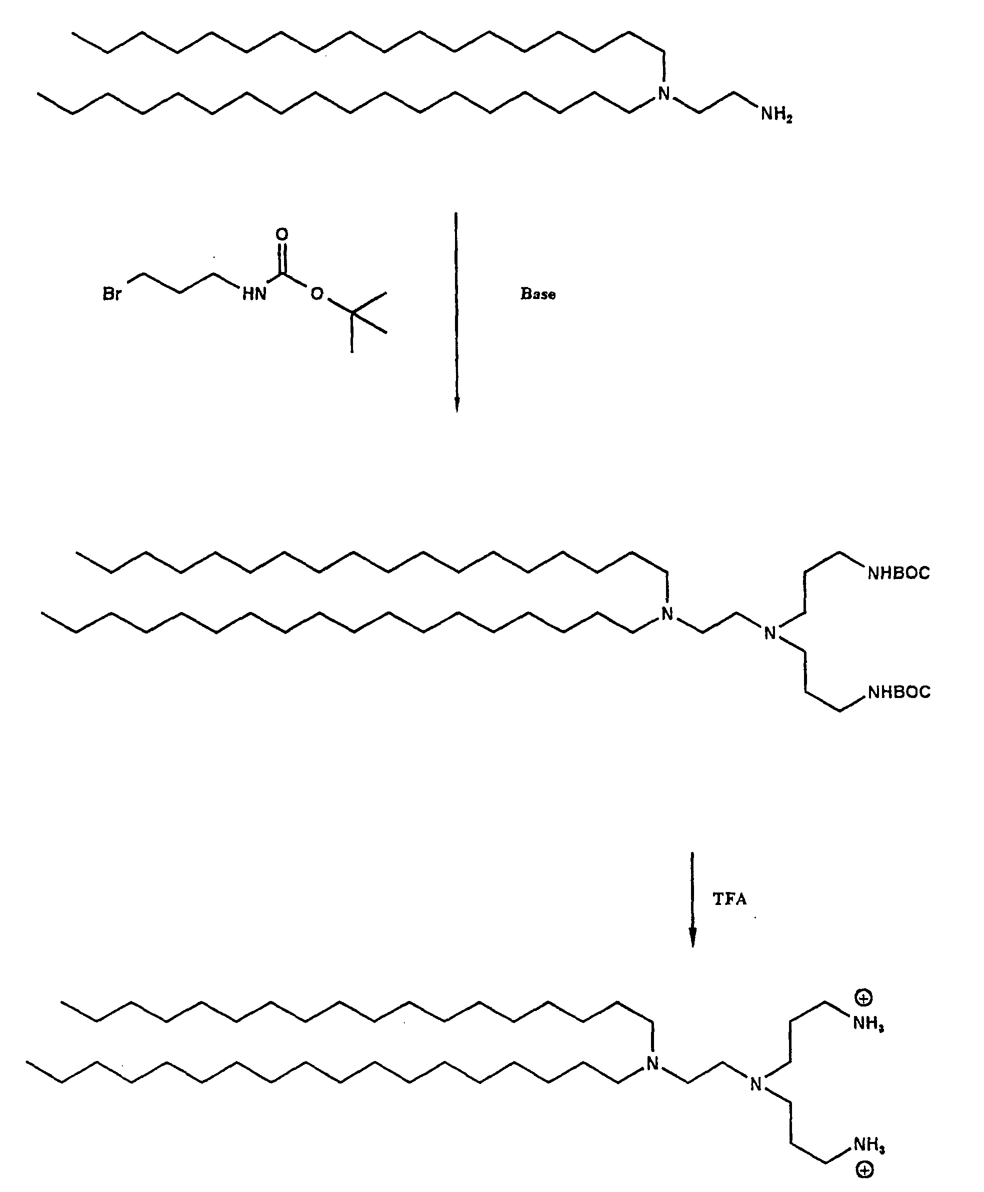

Durch Wahl dem Fachmann an sich bekannter geeigneter Bedingungen, wie Temperatur, Zeit, stöchiometrisches Verhältnis etc. können die Polyaminkopfgruppen verlängert oder auch verzweigte Polyaminkopfgruppen erzeugt werden. Dabei besteht die Möglichkeit, die Kopfgruppe einzeln zu erzeugen und anschließend an den lipophilen Rest zu kuppeln oder schon gekuppelte Kopfgruppen am Molekül zu erweitern. Z.B. kann nach folgendem an sich bekanntem Reaktionsschema, das im übrigen auch im vorigen Beispiel enthalten ist, vorgegangen werden:

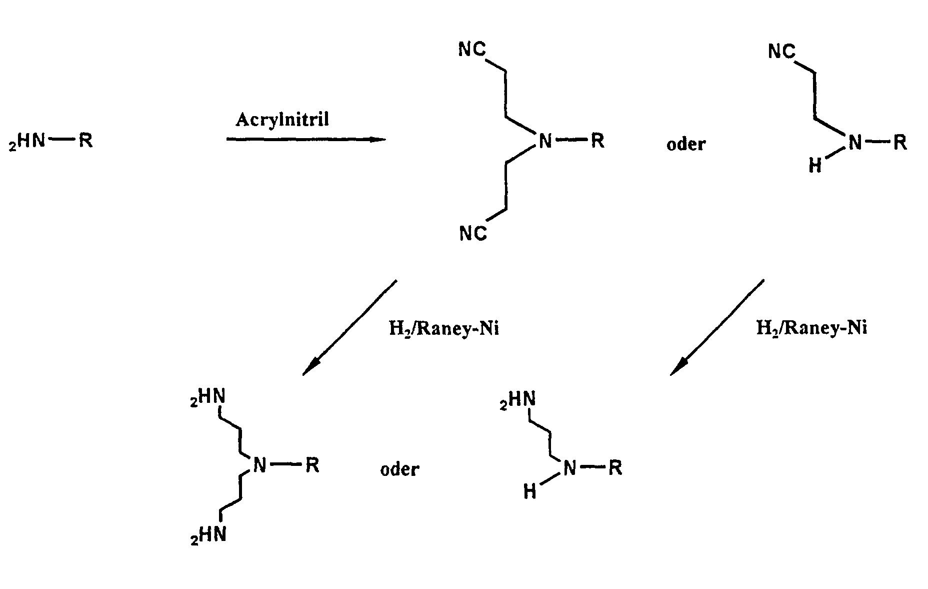

Als explizites Beispiel soll die Umsetzung von Omithin mit Acrylnitril dienen. Über die Menge an Acrylnitril und der richtigen Wahl der Reaktionstemperatur kann das gewünschte Produkt als Haupmenge erhalten werden. Die Hydrierung der Nitrile zu den Aminen und deren Schutz durch die BOC-Schutzgruppe werden nach J. P. Behr et al. Proc. Natl. Acad. Sci., USA, 86, pp. 6982-6986, 1989 analog der Synthese von Tetra-BOC-Carboxyspermin durchgeführt.

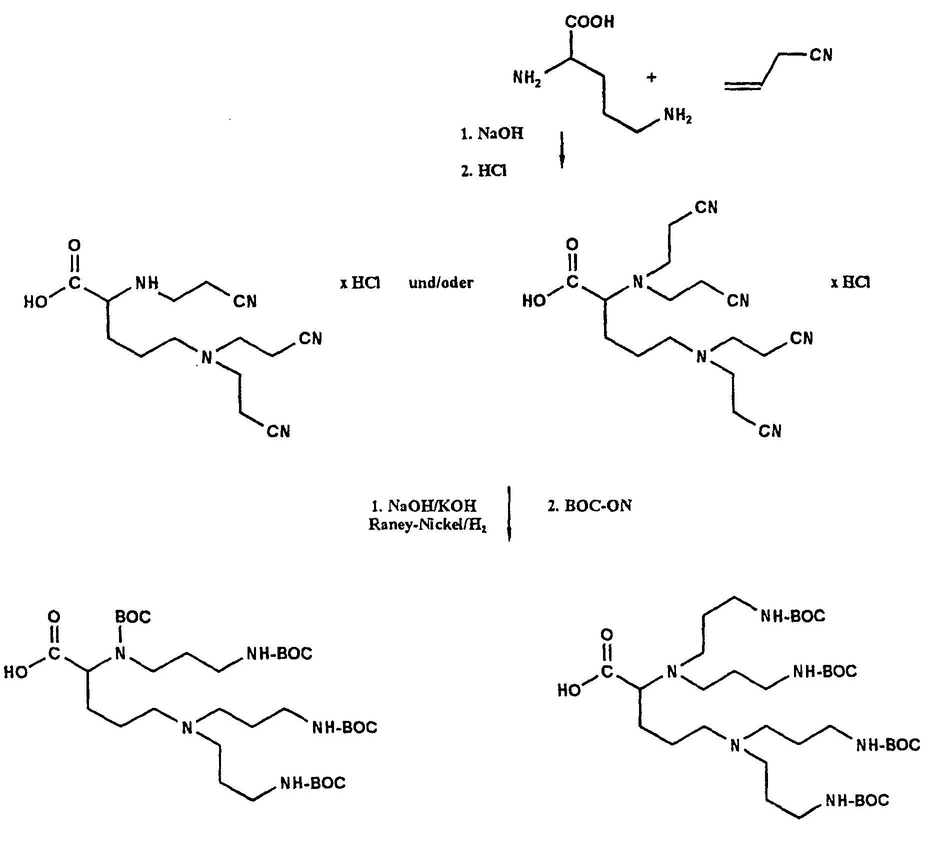

Eine weitere Möglichkeit zur Verlängerung und Verzweigung der Polyaminkopfgruppen besteht in der Alkylierung von Aminen durch N-(tert.-Butyloxycarbonyl)-3-brompropylamin:

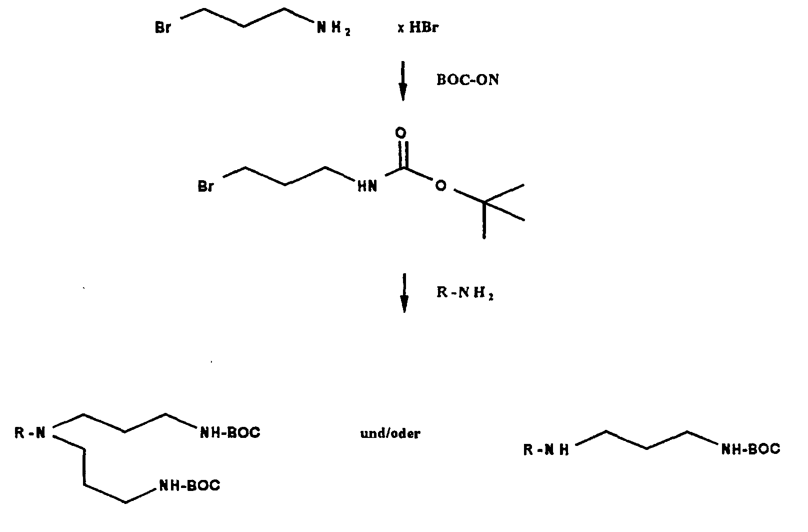

Allgemein können sämtliche aufgeführten Lipide mit den in den vor- und nachstehenden Beispielen aufgeführten Methoden (Blockieren und Deblockieren von Aminofimktionen mit der BOC-Schutzgruppe, Alkylierung von Aminofunktionen mit Alkylbromiden, Knüpfen von Amidbindungen anolog bekannter Peptidchemie, Cyanoethylierung von Aminofunktionen via Acrylnitril, Hydrierung von Cyanofunktionen zu Aminen) oder mit dem organischen Chemiker vertrauten, allgemein bekannten Methoden (Alkylierung von Alkoholen, Knüpfen von Esterbindungen, Reduktion von Estern zu Ethern bzw von Amiden zu Aminen via Lithiumalanat) erzeugt werden.
Bezüglich der Herstellung der Verbindungen, die die zwei zuletzt beschriebenen Spacer enthalten, sei auf WO 9802191 verwiesen. Hier wird beschrieben, wie Ester und Amide mit Lithiumalanat zu Ethem und Aminen reduziert werden können, ohne die BOC-Schutzgruppe anzugreifen. Auf diesem Wege resultieren solche Verbindungen aus den entsprechenden Vorläufern.
Die erfindungsgemäßen Verbindungen können als solche in wässriger oder wässriger Puffer-oder ethanolischer Lösung angewendet werden. Es können jedoch auch Liposomen (oder Micellen) mit den oben genannten Lipiden allein, oder in Kombination mit anderen Lipiden wie zum Beispiel Cholesterol, Cholesterylamin, Dioleoylphosphatidylethanolamin (DOPE) oder Dioleoylphosphatidylcholin (DOPC) formuliert werden.
Um die Transfektionseffizienz der erfindungsgemäßen Lipide zu steigern, besteht die Möglichkeit, Zellen in Gegenwart von Substanzen zu transfektieren, die die enzymatische Aktivität in den Lysosomen inhibieren, sogenannte lysosomatotrope Substanzen, wie zum Beispiel Chloroquin oder von Viren abgeleitete lysosomatropisch wirkende Proteine.
Weiter besteht die Möglichkeit, sogenannte Enhancer in Kombination mit den kationischen Lipiden einzusetzen. Enhancer verstärken die Wirkung der kationischen Lipide. Dabei gibt es Enhancer, die selbst keine transfizierenden Eigenschaften besitzen, und solche, die auch ohne kationische Lipide zur Transfektion verwendet werden können. Im ersten Fall kommt es zu einer einfachen Verstärkung (Amplifikation) der Transfektionseffizienz der kationischen Lipide. In letzterem Fall zeigt sich in kombinierter Anwendung eine Transfektionseffizienz, die größer ist als bei Einzelanwendung. Die Kombinationswirkung kommt daher durch einen Synergieeffekt zustande. Enhancer entfalten ihre Wirkung in der Regel durch eine Vorkondensation der zu transfizierenden DNA oder sie Erhöhen die Permeabilisierbarkeit der zu durchdringenden Zellmembran. Beispiele für Enhancer sind Polyethylenimin, Transferrin, Protaminsulfat, Polyhistone, fusogene Peptide, Virushüllen, virale Oberflächenpeptide, replikationsdefiziente Viren usw.
Die erfindungsgemäßen Lipopolyamine sind bei physiologischem pH positiv geladen und können daher mit negativ geladenen Makromolekülen, insbesondere mit DNA und verwandten Stoffklassen stabile Aggregate bilden. Die mit den Lipopolyaminen bemäntelten und positivierten Makromoleküle wechselwirken mit der negativ geladenen Zellmembran auf eine Weise, die zu einer Einschleusung der Makromoleküle in die Zelle führt. Dabei sind in vitro als auch in vivo Anwendungen möglich.
Im Vergleich zur zielgerichteten rezeptorvermittelten Endocytose (Wu et al., J. Chem. 262, 4429-4432, 1987), in denen Polykationen, welche auch DNA-Binder genannt werden (zum Beispiel Polylysin etc.) an sogenannte Internalisierungsfaktoren (zum Beispiel bestimmte Glykoproteine) gebunden sind, die zielgerichtet an Oberflächenrezeptoren bestimmter Zellen binden, besitzen die erfindungsgemäßen Lipide, bzw. deren Liposomenformulierungen keine Zellspezifität. Eine zielgerichtete Lieferung durch die erfindungsgemäßen Lipide bzw. deren Liposomenformulierungen kann dadurch erreicht werden, daß die Ladungen von Lipiden und den zu transportierenden Biomolekülen ausgeglichen werden und zusätzlich an das Aggregat zwischen Biomolekül und Lipiden Internalisierungsfaktoren angebracht werden. Dies kann zum Beispiel durch Liposomenformulierung mit Colipiden erreicht werden, falls die Colipide als Kopfgruppe solche Internalisierungsfaktoren tragen. Eine andere Möglichkeit ist ein auf Ladungsneutralität abgestimmtes Aggregat aus Biomolekül, Lipiden und Internalisierung-faktoren. Sogenannte Internalisierungsfaktoren sind Transferrin, Galactose, Mannose, Mannose-6-phosphat, Asialglycoprotein, Conalbumin, Lectine, Transcobalamin, α-2-Makroglobulin, Biotin, Folat, mannosylierte Glycoproteine. Weitere sind in EP 0535576, EP 0544292, WO 9421808 zu entnehmen, die hierin unter Bezugnahme aufgenommen werden.
Analog wie Internalisierungsfaktoren, können auch zellspezifische Antikörper eingesetzt werden.
Die erfindungsgemäßen Verbindungen können zu Therapiezwecken eingesetzt werden. Insbesondere können solche Verbindungen für die Gentherapie von zum Beispiel Cystischer Fibrose, Muskeldystrophie, Phenylketonurie, Ahomsirupkrankheit, Propionazidämie, Methylmalonazidämie, Adenosindeaminasemangel, Hypercholesterinämie, Hämophilie und β-Thalassämie genutzt werden. Gentherapeutische Behandlungsmethoden sind weiters interessant, wenn Hormone, Wachstumsfaktoren, Cytotoxine oder immunomodulierend wirkende Proteine im Organismus synthetisiert werden sollen. Für die oben genannten Zwecke können DNA-Fragmente mittels dieser Lipide in Zellen gebracht werden, in denen diese DNA die gewünschte Wirkung entfaltet. Die gewünschte Wirkung kann der Ersatz fehlender oder defekter DNA-Bereiche oder die Inhibition von DNA-Bereichen ( zum Beispiel Antisense-DNA / RNA), die die Erkrankung auslösen, im erkrankten Zelltyp sein. Auf diese Weise können tumorunterdrückende Gene in der Krebs-Therapie eingesetzt werden oder durch die Einführung cholesterolregulierender Gene ein Beitrag zur Vorbeugung von Herz- und Blutgefäßkrankheiten leisten. Weiter kann DNA, welche Ribozyme kodiert, oder Ribozyme selbst in erkrankte Zellen eingeschleust werden. Die Translation jener DNA erzeugt aktive Ribozyme, die an spezifischen Stellen m-RNA katalytisch spalten und auf diese Weise die Transkription verhindern. Auf diese Weise kann zum Beispiel virale m-RNA spezifisch gespalten werden, ohne eine andere zelluläre m-RNA in Mitleidenschaft zu ziehen. Der Vermehrungszyklus von Viren (z.B. HIV, Herpes, Hepatitis) kann auf diesem Wege unterbrochen werden.
Auch in der Krebstherapie spielt Transfektion zur Herstellung von Krebsvakzinen eine immer größer werdende Rolle. Damit ist dieses Feld auch mögliches Anwendungsgebiet fiir die erfindungsgemäßen Verbindungen.
Eine weitere Anwendung können solche Lipide zum Beispiel in Impfverfahren finden, die auf der Basis der Expression von DNA, welche immunogene Peptide kodiert, im Körper von Mensch und Tier funktionieren. Dazu werden Lipid / DNA-Komplexe als Impfstoffe benutzt. Die Einschleusung der DNA in die Körperzellen führt zur Expression des immunogenen Peptids und löst somit die Immunantwort aus.
Abgesehen von DNA können auch andere Makromoleküle, wie zum Beispiel PNA, Peptide, Peptoide oder Proteine, in Zellen eingeschleust werden. Zu diesem Zweck können sie mit den erfindungsgemäßen Lipopolyaminen als solche bemäntelt werden oder in Liposomen, welche als Komponente die erfindungsgemäßen Lipopolyamine enthalten, eingeschlossen oder an deren Oberfläche adsorbiert werden, falls eine negative Nettoladung vorhanden ist. Bringt man derartige Aggregate mit Zellen in Kontakt, so findet ein Transport dieser Moleküle durch die Zellwand statt. Therapeutische Peptide haben einen günstigen Einfluß auf zahlreiche Erkrankungen. Solche Peptide oder Proteine sind zum Beispiel Lymphokine, Interleukine, Tumore Nekrose Faktoren oder Interferone, weiterhin Wachstumsfaktoren, Gewebeplasminogenaktivator, Faktor-VIII:c, Granulocyten-Makrophagen-Kolonie-stimulierender Faktor, Erythropoietin, Insulin, Calcitonin, Thymidinkinase und andere. Auch toxische Peptide wie Ricin, Diphteriatoxin und andere können therapeutisch auf diese Weise gewinnbringend eingesetzt werden. Peptoide können erfolgreich als Peptidanaloga zur Verhinderung eines schnellen enzymatischen Abbaus im Körper eingesetzt werden.
Die erfindungsgemäßen Lipide werden aufgrund ihrer positiven Ladung vorwiegend dazu eingesetzt, negativ geladene Moleküle wegen ihrer negativen Ladung zu komplexieren und in Zellen einzuschleusen. Durch sogenannte "self assembling systems" können jedoch auch positiv geladene Moleküle transportiert werden, in dem zunächst negativ geladene Liposomen mit diesem positiv geladenen Molekülen komplexiert werden. Wählt man die Verhältnisse so, daß eine negative Nettoladung verbleibt, können diese Komplexe mit diesen erfindungsgemäßen Lipopolyaminen als solche oder in Form von Liposomen positiviert werden, in dem die gegensätzlich geladenen Komponenten in Kontakt gebracht werden. Die resultierenden positiv geladenen Gesamtkomplexe werden von den Zellen aufgenommen.
Weitere Anwendungsmöglichkeiten für kationische Lipide sind den Veröffentlichungen WO 9011092, WO 9116024, WO 9303768, Science 258, 744-746, 1992 zu entnehmen, die hierin unter Bezugnahme aufgenommen werden.
Beispiele für derzeit als therapeutisch aussichtsreich anzunehmende Sequenzen genetischen Materials sind in der Übersicht von F.W.Anderson, Science 256, 808, 1992 entnehmbar, die ebenfalls hierin unter Bezugnahme aufgenommen werden.

### Beispiele:

### Bezugsquellen:

1.Plasmide: pCMV<Sport>β-Gal; Gibco BRL, Life Technologies
2. β-Galaktosidase-Assay-Kit, Stratagene
3. Tetra-BOC-Carboxyspermin wurde nach J.-P. Behr et al. Proc. Natl. Acad. Sci., USA, 86, pp. 6982-6986, (1989) synthetisiert.
4. Lipofectamin™; Gibco-BRL : Life Technologies Inc.
5. N-tert.-Butyloxycarbonylethylendiamin, Aldrich.
6. O-(tert.-Butoxycarbonyl)-phenylglyoxyl-säurenitriloxim (BOC-ON); Aldrich.
7. N'-(3-Dimethylaminopropyl)-N-ethylcarbodiimid Methojodid (EDC), Aldrich.
8. 1-Hydroxy-1H-benzotriazol (HOBT), Aldrich.
9. 4-Methylmorpholin, Diisopropylethylamin (DIPEA), Triethylamin, Aldrich.
10. Trifluoressigsäure (TFA), Aldrich.
11. Dulbecco's modified Eagels Medium (DMEM), Gibco-BRL : Life Technologies Inc.
12. Fötales Kälberserum (FCS), Biochrom.
13. Penicillin, Streptomycin, SIGMA.

### Beispiel 1: Transfektionsreagenz 1

### Synthese von N-tert.-Butyloxycarbonyl-N',N'-dioctadecylethylendiamin

646 mg (1.94 mmol, MG 333.4) Octadecylbromid, 135 mg ( 0.84 mmol, Mg 160.22) N-tert.-Butyloxycarbonylethylendiamin (BOC-ethylendiamin) und 332 µl (1.94 mmol, MG 129.25, d 0.755) Diisopropylethylamin (DIPEA) werden in 10 ml Acetonitril gelöst und über Nacht unter Rückfluß gekocht. Nach Zugabe von 100 ml Ether wird mit Wasser gewaschen. Die organische Phase wird mit Magnesiumsulfat getrocknet und die Lösungsmittel abgezogen. Das schon relativ saubere Produkt wird durch Flashchromatographie gereinigt.
C₄₃H₈₈O₂N₂
   MG 664.12
   R_{f}= 0.44 (9:1 v/v Chloroform/Methanol)
   MS (FAB): 665.7 (M +1)
¹H- NMR (CDCl₃):
   0.88 (tr, 6 H, CH₃); 1.26 ("s", 60 H, CH₂); 1.40 (m, 4 H, N-CH₂CH₂); 1.44 (s, 9 H, CH_{3BOC}); 2.40 (tr, 4 H, N-CH₂); 2.50 (tr, 2 H, N-CH₂); 3.15 (m, 2 H, OCO-N-CH₂).
¹³C- NMR (CDCl₃):
   13.1 (CH₃); 22.7, 27.0, 25.5 (CH₂); 28.5 (CH_{3BOC}); 29.4, 29.6 29.7, 29.7 (CH₂); 53.2 (N-CH₂); 54.0 (N- CH₂); 78.9 (C_{qBOC}); 156.1 (OCO).
Ausbeute: 390 mg (69 %)

### Synthese von N-[Tetra-tert.-butyloxycarbonyl-[(2,5-bis(3-aminopropyl)amino)-1-oxopentyl]]-N',N'-dioctadecylethylendiamin

157 mg (0.236 mmol, MG 665.12) N-tert.-Butyloxycarbonyl-N',N'-Dioctadecylethylendiamin werden in 4 ml eines 1:3 v/v Gemisches aus Trifluoressigsäure (TFA) und Methylenchlorid aufgenommen. Nach 2-3 h wird abrotriert und 0.5 h am Hochvakuum getrocknet. Anschließend wird in 6 ml eines 1:1 v/v Gemisches aus DMF und Methylenchlorid aufgenommen und mit 300 µl 4-Methylmorpholin versetzt. Anschließend werden 152.6 mg (0.236 mmol, MG 646.8) Tetra-BOC-Carboxyspermin in 2 ml eines 1:1 v/v Gemisches aus Dimethylformamid (DMF) und Methylenchlorid gelöst und zugegeben. Weiterhin werden 45 mg (0.330 mmol) 1-Hydroxy-1H-benzotriazol (HOBT) und 98 mg (0.330 mmol) N'-(3-Dimethylaminopropyl)-N-ethylcarbodiimid Methojodid (EDC) zugegeben und 50 h bei RT gerührt. Die Lösungsmittel werden abrotiert und der Rückstand in Methylenchlorid aufgenommen. Danach wird mit Wasser gewaschen, die organische Phase mit Natriumsulfat getrocknet und abrotiert. Das Rohprodukt wird über Flashchromatographie gereinigt.
C₅₁H₁₃₆N₆O₉
   MG 977.65
   R_{f} = 0.40 (9: 1 v/v Methylenchlorid/Methanol)
¹H- NMR (CDCl₃):
   0.88 (tr, 6 H, CH₃); 1.25 ("s", 64 H, CH₂); 1.44 (m, 36 H, CH_{3BOC}); 1.67 (m, 8 H, Spermin: CH₂CH₂CH₂); 2.36 (mr, 4 H, Stearyl: N-CH₂); 2.51 (m, 2 H, Ethylendiamin: N-CH₂); 3.1-3.25 (m, 10 H, OC-N-CH₂).
Ausbeute: 135 mg (59 %)

### Synthese von N-[2,5-Bis((3-aminopropyl)amino)-1-oxopentyl]-N',N'-dioctadecylethylen-diamin (TFA-Salz)

70 mg N-[Tetra-tert.-butyloxycarbonyl-[(2,5-bis(3-aminopropyl)amino)-1-oxopentyl]]- N',N'-dioctadecylethylendiamin werden in 2 ml eines Gemisches aus Methylenchlorid und Trifluoressigsäure (3:1 v/v) gelöst und 1.5 h bei RT gerührt. Danach wird abrotiert, in 10 ml eines Gemisches aus Wasser/ tert.-Butanol (1:1 v/v) aufgenommen und lyophilisiert.
C₅₃H₁₀₄ON₆ (x 4 CF₃COOH)
MG 793.52 (+ 4 x 114 = 1249.52)
Ausbeute: 100%

### Beispiel 2: Transfektionreagenz 2

### Synthese von N',N'-Bis-(2-cyanoethyl)-N-tert.-butyloxycarbonylethylendiamin

500 mg (3.12 mmol, MG 160.22) N-tert.-Butyloxycarbonylethylendiamin werden in 5 ml Wasser gelöst und 0.65 ml (9.8 mmol, MG 53.06) Acrylnitril zugegeben. Danach wird 7 Tage bei Raumtemperatur gerührt. Danach wird mit Essigester überschichtet, ausgeschüttelt und die organische Phase abgetrennt. Nach Trocknen über Natriumsulfat wird abrotiert.
C₁₃H₂₂O₂N₄
MG 266.34
R_{f} = 0.69 (9:1 v/v Chloroform/Ethanol)
Ausbeute: 660 mg (80%)

### Synthese von N',N'-Bis-(2-cyanoethyl)-N,N-dioctadecylethylendiamin

660 mg (2.48 mmol, MG 266.34) N',N'-Bis-(2-cyanoethyl)-N-tert.-butyloxycarbonylethylendiamin werden in 10 ml eines 1:3 v/v Gemisches aus Trifluoressigsäure und Methylenchlorid aufgenommen. Nach 2-3 h wird abrotriert und 0.5 h am Hochvakuum getrocknet. Danach wird aus Butanol/Wasser (1:1 v/v) lyophilisiert. Das erhaltene Trifluoracetat wird in 40 ml trockenen Acetonitril zusammen mit 2.43 g (7.28 mmol, MG 333.4) Octadecylbromid und 2.23 ml (1.68 g, 13.02 ml, MG 129.25, d 0.755) Diisopropylethylamin (DIPEA) gelöst und 24 Stunden unter Rückfluß gekocht. Das Rohprodukt wird über Flashchromatographie gereinigt.
C₄₄H₈₆N₄
   MG 671.16
   R_{f} = 0.42 (9:1 v/v Methylenchlorid/Methanol)
¹H- NMR (CDCL₃):
   0.88 (tr, 6 H, CH₃); 1,26 ("s", 60 H, CH₂); 1.42 (m, 4 H, N- CH₂CH₂) 2.42 (m, 4 H, N- CH₂); 2.48 (tr, 4 H, CH₂-CN); 2.50 (m, 2 H, N- CH2); 2.50 (m, 2 H, N- CH₂); 2.92 (tr, 4 H, N-CH₂).
Ausbeute: 330 mg (20%)

### Synthese von N',N'-Bis-(3-aminopropyl)-N,N-dioctadecylethylendiamin (TFA-Salz)

2.5 g Raney-Nickel werden zu 50 ml Wasser gegeben. Dazu wird unter Rühren 4.5 g Natriumhydroxid so schnell zugegeben, daß das Wasser gerade nicht überkocht und anschließend eine halbe Stunde gerührt. 320 mg (0.48 mmol, MG 671.2) N',N'-Bis-(2-cyanoethyl)-N,N-dioctadecylethylendiamin werden in 500 ml 0.5 M Kaliumhydroxidlösung (in EtOH/Wasser 95:5 v/v) aufgenommen. Das aktivierte Raney-Nickel wird vom Überstand durch abdekantieren getrennt und zur Eduktlösung gegeben. Danach wird 24 Stunden an einer Hydrieranlage hydriert. Nach Einrotieren des Lösungsmittels wird der Rückstand in 5 ml Methylenchlorid gelöst. Dazu werden 500 mg (2.02 mmol, MG 246.27) O-(tert.-Butoxycarbonyl)-phenylglyoxyl-säurenitriloxim (BOC-ON) gegeben und bei Raumtemperatur 24 Stunden gerührt. Danach wird einrotiert. Das Rohprodukt wird über Flashchromatographie gereinigt. Danach wird das Produkt in 10 ml eines 1:3 v/v Gemisches aus Trifluoressigsäure und Methylenchlorid aufgenommen. Nach 2-3 h wird abrotriert und 0.5 h am Hochvakuum getrocknet. Zuletzt wird aus Butanol/Wasser (1:1 v/v) lyophilisiert.
C₄₄H₉₄N₄ x 4 CF₃COOH
   MG 679.21 (+4 x 114 = 1135.21)
   MS (ESI): 680.2
Ausbeute: 250 mg (58% bezogen auf Nitril)

### Beispiel 3: Verzweigte Kopfgruppen:

### Synthese von N-(tert.-Butyloxycarbonyl)-3-brompropylamin

500 mg (2.28 mmol; MG 218.94) 3-Brom-1-propylamin Hydrobromid (Aldrich) werden zusammen mit 1.13 (4.59 mmol; MG 246.27) O-(tert.-Butoxycarbonyl)-phenylglyoxyl-säurenitriloxim (BOC-ON) und 0.65 ml (0.46 g; 4.6 mmol) Triethylamin in einem Gemisch aus Aceton und Wasser (1:1, v/v) gelöst. Nach 2 Stunden Rühren wird eingeengt und in Methylenchlorid aufgenommen. Die organische Phase wird mit Wasser gewaschen, mit Natriumsulfat getrocknet und einrotiert. Das Rohprodukt wird durch Flashchromatographie gereinigt.
C₈H₁₆O₂NBr
   MG 238.31
   R_{f}= 0.84 (9:1 v/v Methylenchlorid/Methanol)
¹H- NMR (CDCl₃):
   1.36 (br s, 9 H, CH_{3BOC}); 1.98 (m, 2 H, C-CH₂-C); 3.19 (m, 2 H, Br-CH₂); 3.38 (m, 2 H, N-CH₂); 4.90 (br s, 1 H, N-H).
¹³C- NMR (CDCl₃):
   28.2 (CH₃); 30.6,32.6,38.8 (CH₂); 79.1 (C_{q}); 155.8 (C=O).
Ausbeute: 250 mg (46 %)

### Synthese von N',N'-Bis-(3-aminopropyl)-N.N-dioctadecylethylendiamin (TFA-Salz)

200 mg (0.3 mmol, MG 665.12) N-tert.-Butyloxycarbonyl-N',N'-dioctadecylethylendiamin werden in 2 ml eines Gemisches aus Trifluoressigsäure und Methylenchlorid (1:3 v/v) gelöst. Nach zwei Stunden wird abrotiert und 2 Stunden im Hochvakuum getrocknet. Danach wird in 20 ml Chloroform aufgenommen und 1 ml (920 mg, 4.5 mmol, MG 101.15, d = 0.92) 4-Methylmorpholin zugegeben. Anschließend werden 640 mg (2.68 mmol, MG 238.13) N-(tert.-Butyloxycarbonyl)-3-brompropylamin zugegeben. Nach 48 Stunden Kochen unter Rückfluß wird einrotiert und das BOC-geschützte Vorläufer produkt durch Flashchromatograpie gereinigt. Abschließend wird in 2 ml ml eines Gemisches aus Trifluoressigsäure und Methylenchlorid (1:3 v/v) gelöst, einrotiert und lyophilisiert.
C₄₄H₉₄N₄ x 4 CF₃COOH
   MG 679.21 (+4 x 114 = 1135.21)
   MS (ESI): 680.2
Ausbeute: 46 mg (13 %)

### Synthese von N,N',N'-Tris (cyanoethyl)-ornithin

950 mg Natriumhydroxid werden in 20 ml Wasser gelöst. In der Natronlauge werden 2 g (11.86 mmol, MG 168.62) Ornithin Hydrochlorid gelöst. Danach werden 3 ml (45.57 mmol) Acrylnitril zugetropft und 24 Stunden bei Raumtemperatur gerührt. Nach der Zugabe von 1.7 ml 25%iger Salzsäure fällt ein Feststoff aus. Dieser wird nach Kühlung abgenutscht.
C₁₄H₂₁O₂N₅ x HCl
   MG 327.82
   R_{f} = 0.71 (1:1:1 v/v/v Isopropanol/Ethanol/Wasser)
¹H- NMR (D₂O):
   1.58 (m, 2 H, CH₂-CH_{Om}); 1.90 (m, 2 H, CH_{2Om}); 2.61 (m, 6 H, NC-CH₂); 2.86 (tr, 4 H, N-CH₂); 2.98 (tr, 2 H, N-CH₂-CH₂); 3.39 (tr, 2 H, , N-CH₂); 368 (tr, 1 H,CH_{Om}).
Ausbeute: 2.1 g (54 %)

### Synthese von N,N,N',N'-Tetra (cyanoethyl)-ornithin

980 mg Natriumhydroxid werden in 20 ml Wasser gelöst. In der Natronlauge werden 2 g (11.86 mmol, MG 168.62) Ornithin Hydrochlorid gelöst. Danach werden 3.9 ml (59.3 mmol) Acrylnitril zugetropft und 8 Tage bei Raumtemperatur gerührt. Danach werden nochmals 3 ml Acrylnitril zugetropft und weitere 6 Stunden gerührt. Nach der Zugabe von 1.7 ml 25%iger Salzsäure wird das Produkt 3 x mit Essigester ausgeschüttelt. Die organische Phase wird über Natriumsulfat getrocknet und einrotiert.
C₁₇H₂₄O₂N₆ x HCl
   MG 344.4182
   R_{f} = 0.84(1:1:1 v/v/v Isopropanol/Ethanol/Wasser)
¹H- NMR (D₂O):
   1.60 (m, 2 H, CH₂-CH_{Om}); 1.92 (m, 2 H, CH_{2Om}); 2.52 (m, 8 H, NC-CH₂); 2.61 (tr, 4 H, CH_{2Om}); 2.87 (tr, 4 H, , N-CH₂); 3.03 (tr, 4 H, N-CH₂); 3.38 (m, 1 H, CH_{Om}).
Ausbeute: 2.4 g (59 %)

### Beispiel 4:

### Liposomen/Micellenformulierung

Dioleoylphosphatidylethanolamin, Dioleoylphosphatidylcholin, Cholesterol oder Cholesterylamin werden in einem organischen Lösungsmittel (z.B. Chloroform) gelöst. Das Lipid aus Beispiel 1 wird als solches oder in Form seines Salzes, zum Beispiel Trifluoracetat-Salzes in einem organischen Lösungsmittel (z.B. Chloroform) gelöst. Durch Kombination verschiedener Mengen der unterschiedlichen Lösungen werden verschiedene Kompositionen aus Colipiden und kat. Lipid hergestellt. In einem Glaskolben wird mit Hilfe eines Rotationsverdampfers ein dünner Lipidfilm erzeugt. Dieser wird im Hochvakuum von Lösungsmittelresten befreit. Der Film wird mit soviel Wasser oder wässriger Pufferlösung hydratisiert, daß die Konzentration 2 mg Lipid pro ml Lösung beträgt. Danach wird unter Kühlung mit Ultraschall (3 x 15 min) behandelt. Die Formulierung wird durch einem Filter (Porenweite 0.2 µm) sterilfitriert.

### Beispiel 5:

### Lipidlösungen

Die Lipide werden als solches und in Form ihrer TFA-Salze mit oder ohne Colipiden in Ethanol gelöst. Durch Mischen mit Wasser erhält man Lösungen unterschiedlicher Zusammensetzungen.

### Beispiel 6:

### Zellkulturen

Die Zelllinien CV-1, Hela S3 und NIH 3T3 werden in "Dulbecco's-modified Eagle's medium" (DMEM), das 10% fötales Kälberserum (FBS), 2 mM L-Glutamin (Gln), 0.1 mM nicht essentielle Aminosäuren (NEAA), 100 U/ml Penicillin und 100 µg/ml Streptomycin enthält, in einem Inkubator (5% CO₂- Atmosphäre) kultiviert.

### Beispiel 7:

### Transfektion

Die Zellen werden auf einer 12-Well-Mikrotiterplatte mit einer Dichte von 1-1.5 x 10⁵ ausplattiert und über Nacht zu annähernd 60-80 % Konfluenz inkubiert. Um die Zellen in einem "Well" zu transfizieren, werden 1.5 µg von pCMV<Sport>β-Gal in 50 µl serum- und antibiotikafreiem DMEM gelöst. Das kationische Lipid (z.B. 3,6,9,12 µl) als ethanolische Lösung oder Liposomenformulierung wird ebenfalls in 50 µl serum- und antibiotikafreiem DMEM gelöst. Die beiden Lösungen werden in einem Polystyrolbehälter gemischt und für 10-15 min stehengelassen, um die Bildung des Lipid/DNA-Komplexes zu ermöglichen.
Währendessen werden die Zellen einmal mit PBS (phosphate buffered saline) gewaschen. Je nach gewünschten Bedingungen (Transfektion im serumfreien oder serumhaltigen Milieu) werden 0.4 ml antibiotikafreies DMEM mit oder ohne Serum zu den Zellen gegeben. Der DNA/Lipid-Komplex wird direkt zu den Zellen gegeben und im Brutschrank 6 h inkubiert. Danach wird mit serum und antibiotikahaltigem DMEM in der Weise auf 1ml aufgefüllt, daß das Medium eine Endkonzentration von 10 % Serum enthält. Die Zellen werden weitere 20 h kultiviert. Danach wird auf β-Galactosidase nach den Angaben des Herstellers des β-Galaktosidase-Assay-Kits getestet. Das entwickelte O-Nitrophenol wird photometrisch bei 405 nm vermessen.

### Resultate

Folgende Tabelle und folgendes Balkendiagramm zeigen Transfektioneffizienzen am Beispiel von Hela S3 Zellen im Vergleich.
Transfektionsreagenz 1:
   N-[2,5-Bis((3-aminopropyl)amino)-1-oxopentyl]-N',N'-dioctadecylethylen-diamin (TFA-Salz) wurde als Liposomenformulierung mit 25 Mol % Dioleoylphosphatidylethanolamin in wässriger steriler Lösung eingesetzt. Gesamtlipidmenge: 2 mg/ml.
Transfektionsreagenz 2:
   N',N'-Bis-(3-aminopropyl)- N,N-dioctadecylethylendiamin (TFA-Salz) wurde als Liposomenformulierung mit 50 Mol % Dioleoylphosphatidylethanolamin in wässriger steriler Lösung eingesetzt. Gesamtlipidmenge: 2 mg/ml.
Transfektionsreagenz 3:
   Lipofectamin™ (Gibco-BRL : Life Technologies Inc.) wurde vom Hersteller erworben.

| **Absorbtionswerte bei 405 nm:** | | | | | | |
|---|---|---|---|---|---|---|
| | Transfektionsreagenz 1 | | Transfektionsreagenz 2 | | Transfektionsreagenz 3 | |
| | mit Serum | ohne Serum | mit Serum | ohne Serum | mit Serum | ohne Serum |
| 3 µl | 1.61 | 1.92 | 0.63 | 1.65 | 0.81 | 1.93 |
| 6 µl | 2.40 | 2.40 | 0.71 | 0.80 | 0.30 | 1.65 |
| 9 µl | 2.10 | 1.68 | 0.95 | 0.58 | 0.19 | 0.44 |

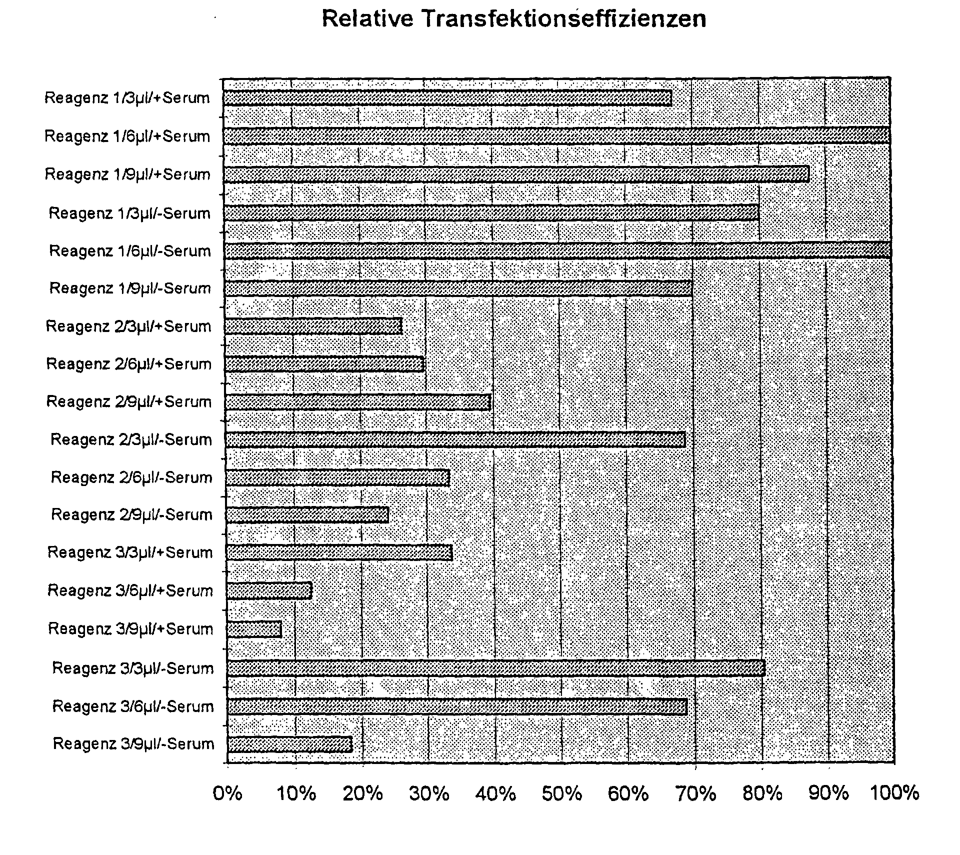

## Patentansprüche

1. Verbindungen der allgemeinen Formel **I**: 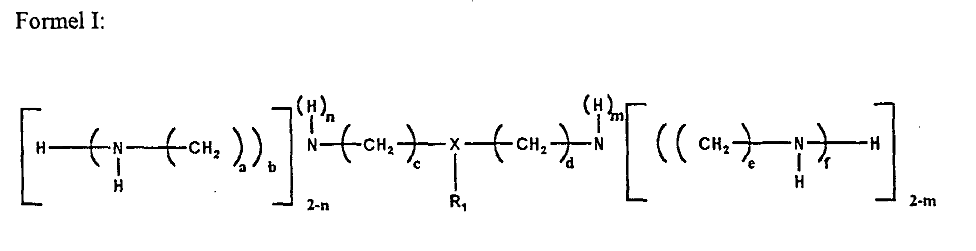 die bei Vorhandensein eines Asymmetriezentrums in der D-,L- oder DL-Form vorliegen, einschließlich ihrer Salze, wobei
R₁ ein lipophiler Rest folgender allgemeiner Formel ist: 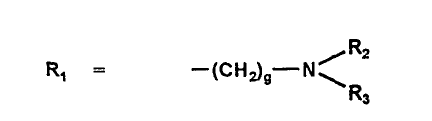 in welcher R₂ und R₃ unabhängig voneinander Dodecyl, Dodecenyl, Tetradecyl, Tetradecenyl, Hexadecyl, Hexadecenyl, Octadecyl, Octadecenyl sind, oder andere Alkylreste, die gesättigt oder ungesättigt, verzweigt oder unverzweigt, fluoriert oder nicht fluoriert sein können, und aus 5, 6, 7, 8, 9, 10, 11, 12, 13, 14, 15, 16, 17, 18, 19, 20, 21, 22, 23, 24, 25, 26, 27, 28, 29 oder 30 Kohlenstoffatomen aufgebaut sind,
und X eine der folgenden Gruppierungen ist: 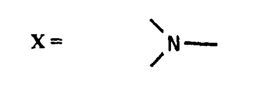 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 0 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 1 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 1 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 2 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder 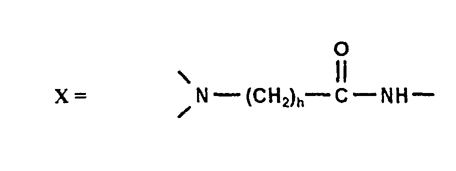 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder
wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder
wobei m = 1 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und h = 0,1,2,3,4,5 oder 6 sein können, oder 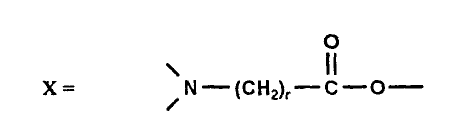 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 and r = 0,1,2,3,4,5 oder 6 sein können,
oder
wobei m = 0 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und r = 0,1,2,3,4,5 oder 6 sein können,
oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und r = 0,1,2,3,4,5 oder 6 sein können,
oder
wobei m = 1 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und r = 0,1,2,3,4,5 oder 6 sein können,
oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und r = 0,1,2,3,4,5 oder 6 sein können,
oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und r = 0,1,2,3,4,5 oder 6 sein können,
oder 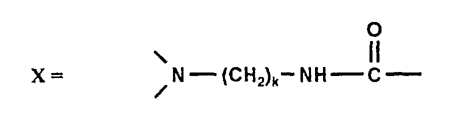 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 0 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 1 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 1 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, k = 0,1,2,3,4,5 oder 6 sein können, oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können,
oder 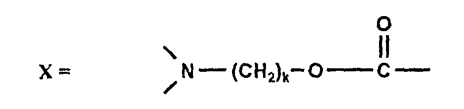 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, k = 0,1,2,3,4,5 oder 6 sein können, oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, k = 0,1,2,3,4,5 oder sein können, oder
wobei m = 1 and n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, k = 0,1,2,3,4,5 oder 6 sein können, oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 , k = 0,1,2,3,4,5 oder 6 sein können, oder
wobei m = 2 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6, k = 0,1,2,3,4,5 oder 6 sein können,
oder 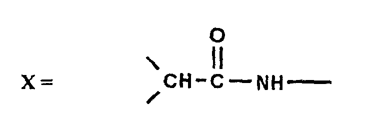 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 1 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder 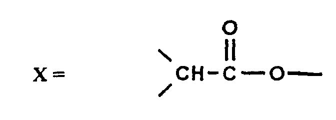 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder 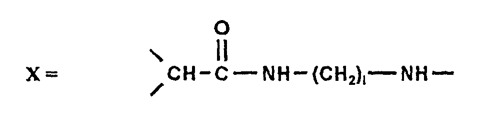 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1 = 0,1,2,3,4,5 oder 6 sein können, oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1= 0,1,2,3,4,5 oder 6 sein können, oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1 = 0,1,2,3,4,5 oder 6 sein können, oder
wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1 = 0,1,2,3,4,5 oder 6 sein können, oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1 = 0,1,2,3,4,5 oder 6 sein können, oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 und 1 = 0,1,2,3,4,5 oder 6 sein können,
oder 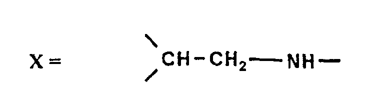 wobei m = 0 und n = 0 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 und n = 2 sind und fiir diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 2 and n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, oder 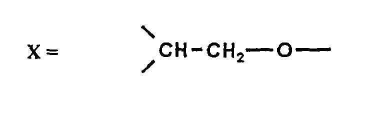 wobei m = 0 und n = 0 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 0 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 and n = 1 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 1 and n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können,
oder
wobei m = 2 und n = 2 sind und für diesen Fall g = 1,2,3,4,5,6,7 oder 8, a = 0,1,2,3,4,5 oder 6, b = 0,1,2,3,4,5 oder 6, c = 0,1,2,3,4,5 oder 6, d = 0,1,2,3,4,5 oder 6, e = 0,1,2,3,4,5 oder 6, f = 0,1,2,3,4,5 oder 6 sein können, mit der Maßgabe, daß in allen vorstehenden Fällen b kleiner oder gleich 1 ist, wenn a = 0 ist und f kleiner oder gleich 1 ist, wenn e = 0 ist, und der Fall ausgeschlossen ist, daß a = b = c = d = e = f = 0.

2. Verbindungen und ihre Salze entsprechend Anspruch 1 mit folgenden Strukturen: 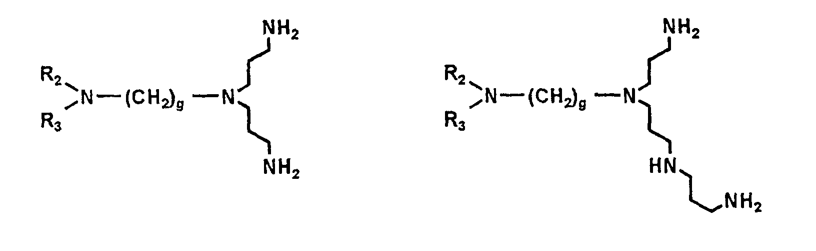 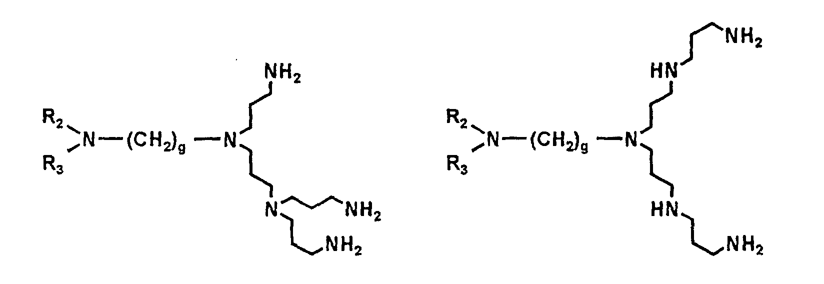 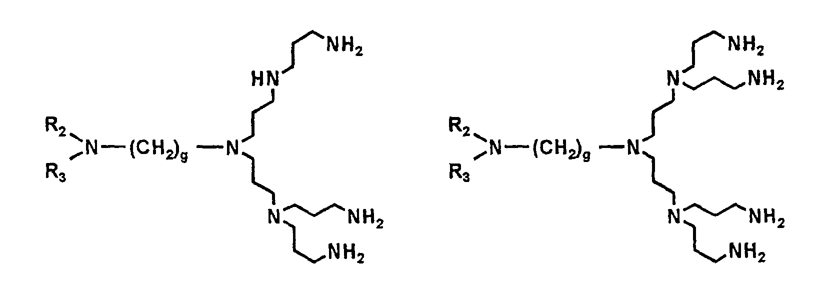 wobei g = 1,2,3,4,5,6,7 oder 8 sein kann und R₂ und R₃ unabhängig voneinander Dodecyl, Dodecenyl, Tetradecyl, Tetradecenyl, Hexadecyl, Hexadecenyl, Octadecyl oder Octadecenyl sein können.

3. Verbindungen und ihre Salze entsprechend Anspruch 1 mit folgenden Strukturen: 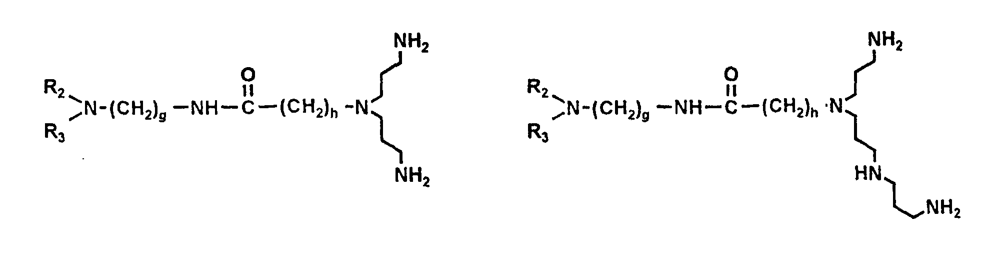 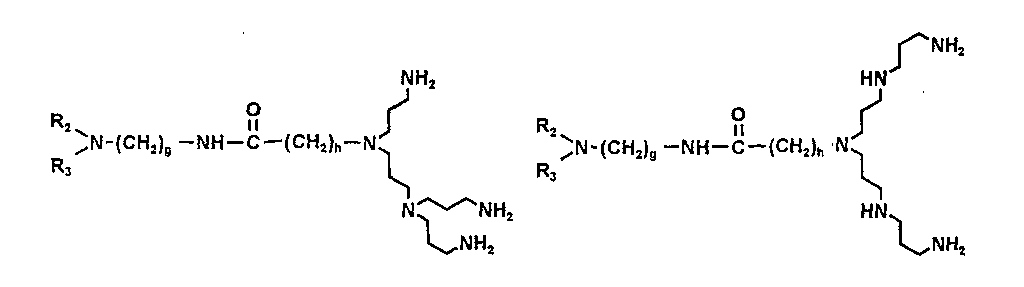 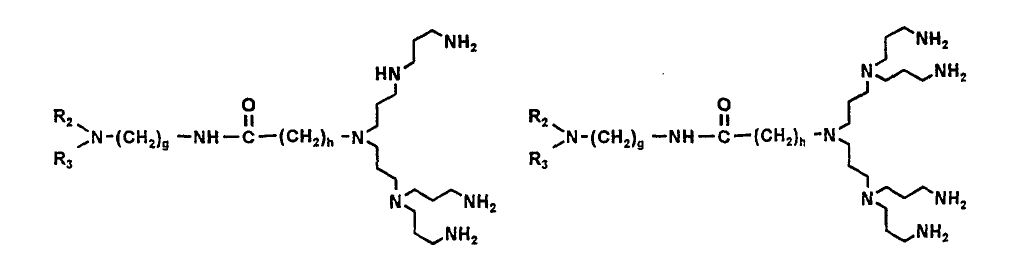 wobei h = 0,1,2,3,4,5 oder 6 und g = 1,2,3,4,5,6,7 oder 8 sein können und R₂ und R₃ unabhängig voneinander Dodecyl, Dodecenyl, Tetradecyl, Tetradecenyl, Hexadecyl, Hexadecenyl, Octadecyl oder Octadecenyl sein können.

4. Verbindungen und ihre Salze entsprechend Anspruch 1 mit folgenden Strukturen: 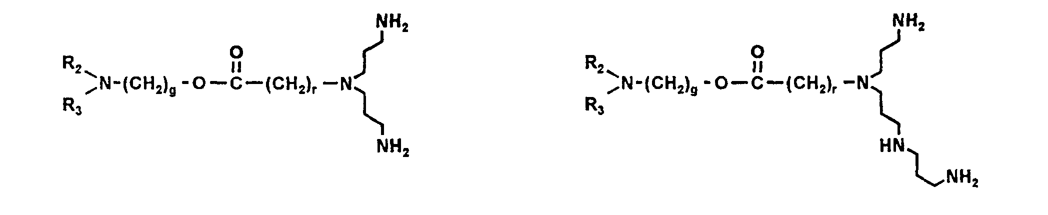 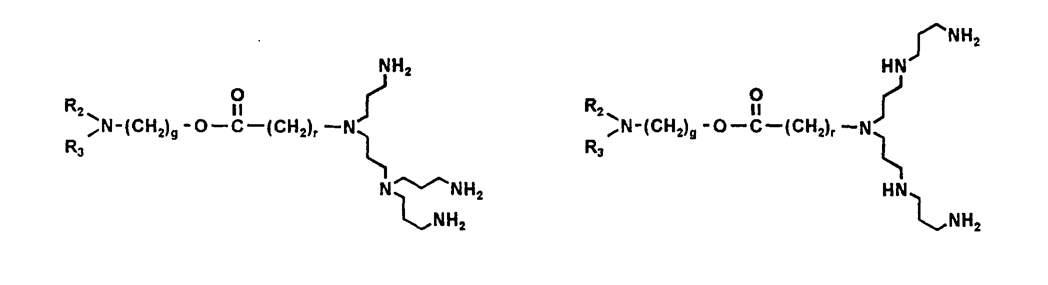 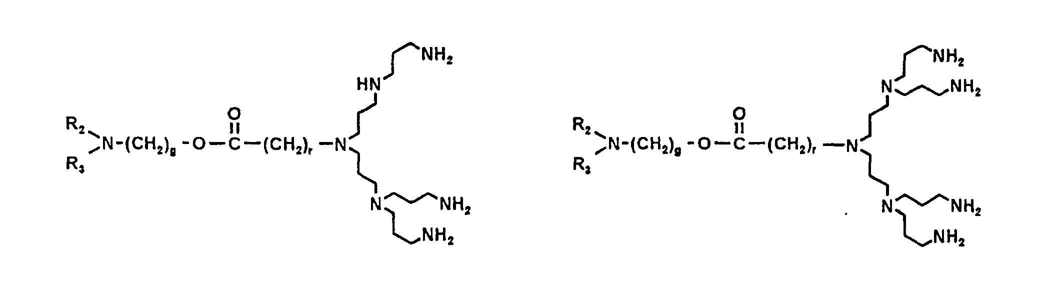 wobei r = 0,1,2,3,4,5 oder 6 und g = 1,2,3,4,5,6,7 oder 8 sein können und R₂ und R₃ unabhängig voneinander Dodecyl, Dodecenyl, Tetradecyl, Tetradecenyl, Hexadecyl, Hexadecenyl, Octadecyl oder Octadecenyl sein können.

5. Verbindungen und ihre Salze entsprechend Anspruch 1 mit folgenden Strukturen: 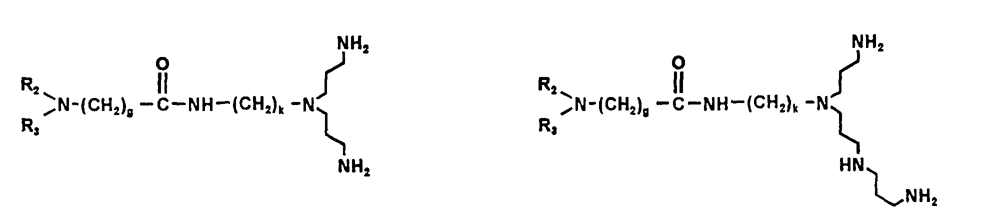 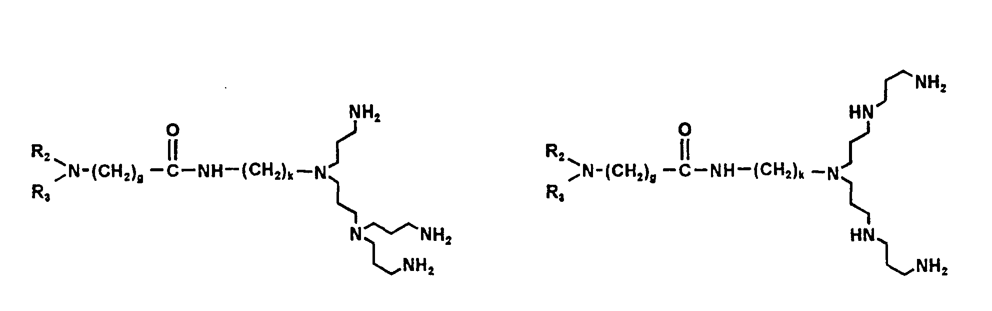 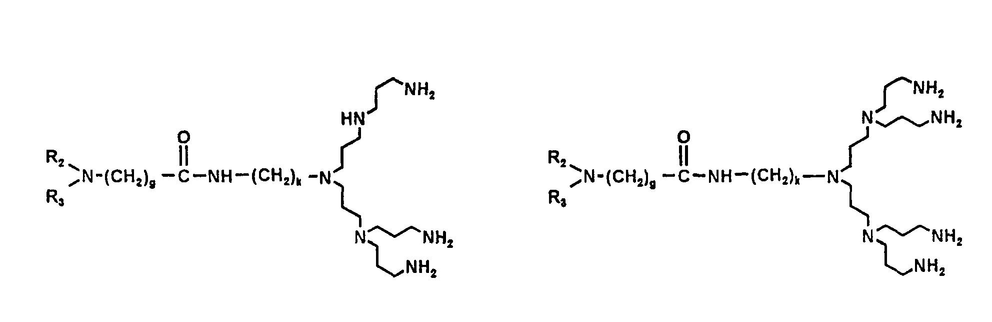 wobei k = 1,2,3,4,5 oder 6 und g = 1,2,3,4,5,6,7 oder 8 sein können und R₂ und R₃ unabhängig voneinander Dodecyl, Dodecenyl, Tetradecyl, Tetradecenyl, Hexadecyl, Hexadecenyl, Octadecyl oder Octadecenyl sein können.

6. Verbindungen und ihre Salze entsprechend Anspruch 1 mit folgenden Strukturen: 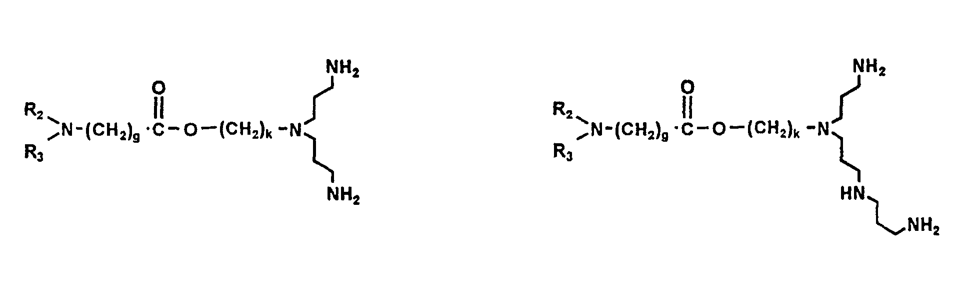 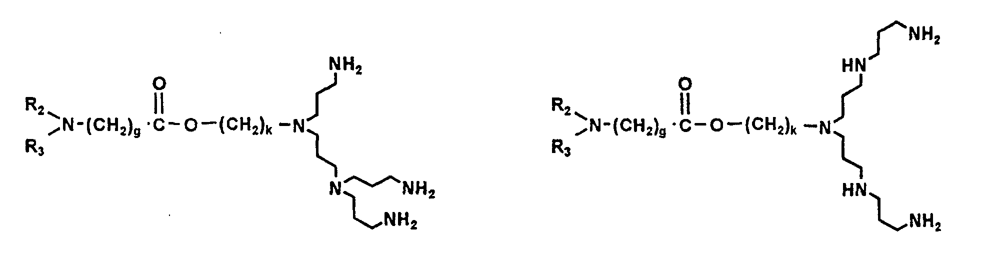 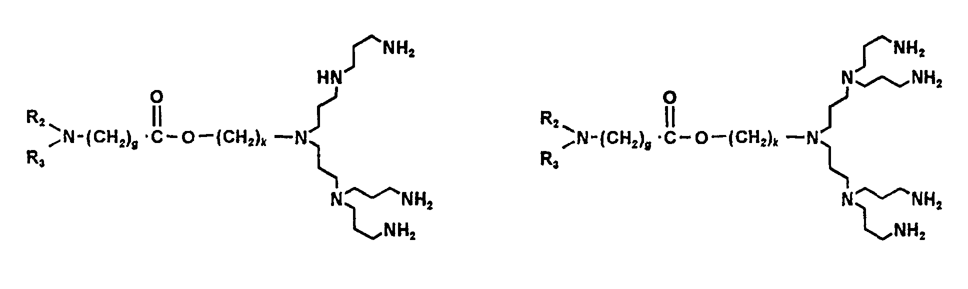 wobei k = 1,2,3,4,5 oder 6 und g = 1,2,3,4,5,6,7 oder 8 sein können und R₂ und R₃ unabhängig voneinander Dodecyl, Dodecenyl, Tetradecyl, Tetradecenyl, Hexadecyl, Hexadecenyl, Octadecyl oder Octadecenyl sein können.

7. Verbindungen und ihre Salze entsprechend Anspruch 1 mit folgenden Strukturen: 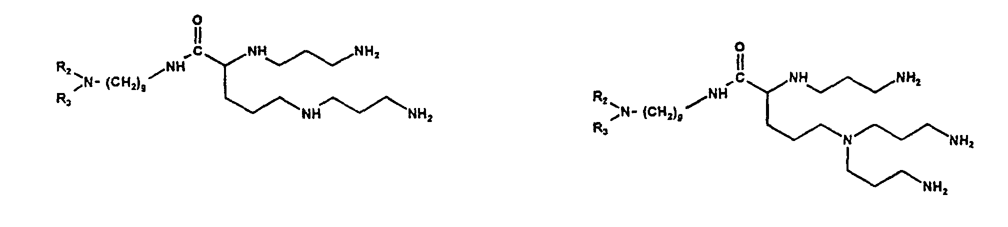 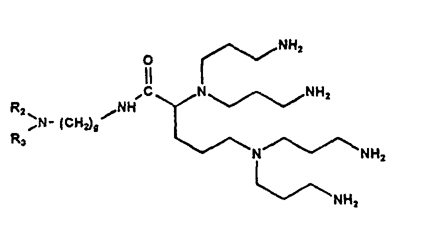 wobei g = 1,2,3,4,5,6,7 oder 8 sein kann und R₂ und R₃ unabhängig voneinander Dodecyl, Dodecenyl, Tetradecyl, Tetradecenyl, Hexadecyl, Hexadecenyl, Octadecyl oder Octadecenyl sein können.

8. Verbindungen und ihre Salze entsprechend Anspruch 1 mit folgenden Strukturen: 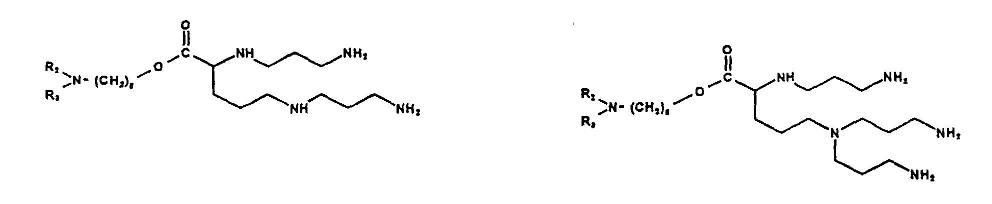 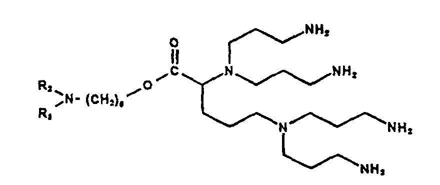 wobei g = 1,2,3,4,5,6,7 oder 8 sein kann und R₂ und R₃ unabhängig voneinander Dodecyl, Dodecenyl, Tetradecyl, Tetradecenyl, Hexadecyl, Hexadecenyl, Octadecyl oder Octadecenyl sein können.

9. Verbindungen und ihre Salze entsprechend Anspruch 1 mit folgenden Strukturen: 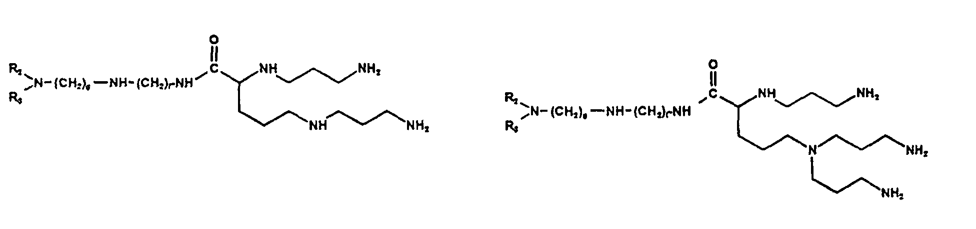 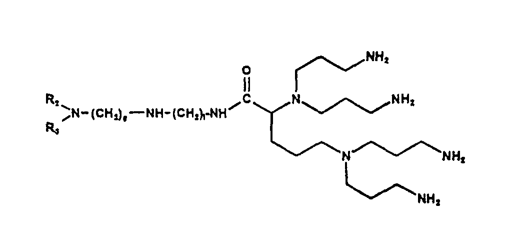 wobei 1 = 0,1,2,3,4,5 oder 6 und g 1,2,3,4,5,6,7 oder 8 sein können und R₂ und R₃ unabhängig voneinander Dodecyl, Dodecenyl, Tetradecyl, Tetradecenyl, Hexadecyl, Hexadecenyl, Octadecyl oder Octadecenyl sein können.

10. Verbindungen und ihre Salze entsprechend Anspruch 1 mit folgenden Strukturen: 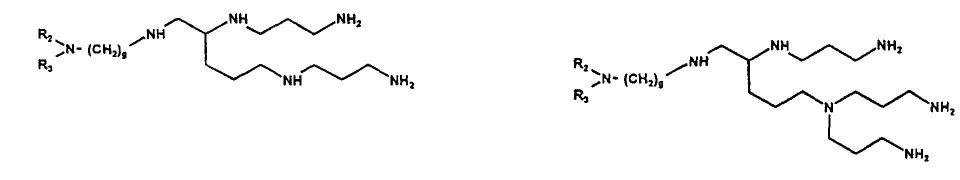 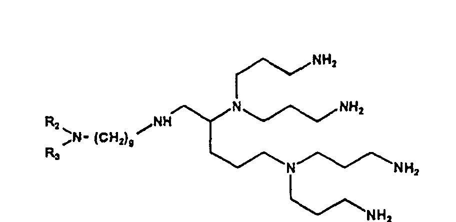 wobei g = 1,2,3,4,5,6,7 oder 8 sein kann und R₂ und R₃ unabhängig voneinander Dodecyl, Dodecenyl, Tetradecyl, Tetradecenyl, Hexadecyl, Hexadecenyl, Octadecyl oder Octadecenyl sein können.

11. Verbindungen und ihre Salze entsprechend Anspruch 1 mit folgenden Strukturen: 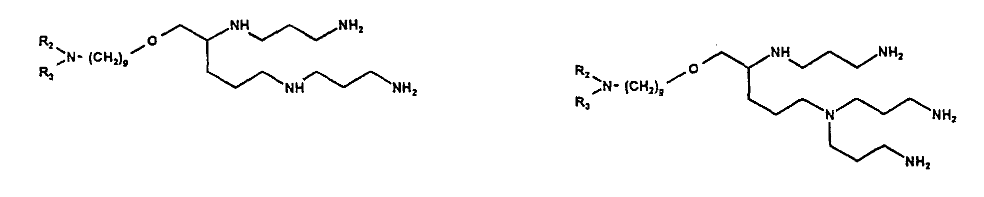 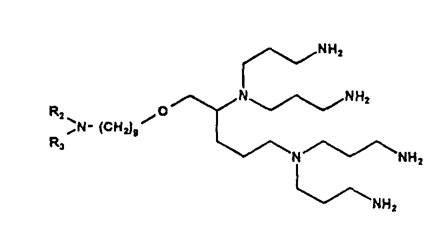 wobei g = 1,2,3,4,5,6,7 oder 8 sein kann und R₂ und R₃ unabhängig voneinander Dodecyl, Dodecenyl, Tetradecyl, Tetradecenyl, Hexadecyl, Hexadecenyl, Octadecyl oder Octadecenyl sein können.

12. Verbindungen und ihre Salze entsprechend Anspruch 1 mit folgenden Strukturen:

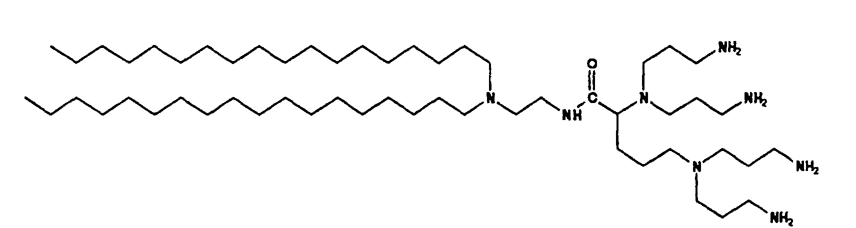

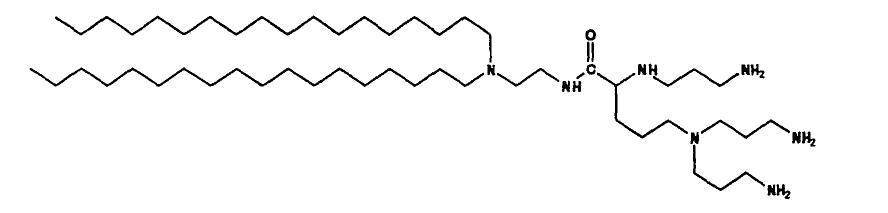

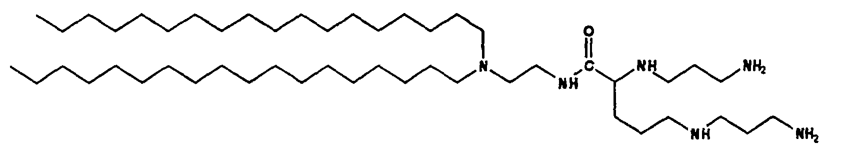

13. Zusammensetzungen, die mindestens eine der Verbindungen der Ansprüche 1-12 mit oder ohne Colipide, wie z.B. Dioleoylphosphatidylethanolamin (DOPE), Dioleoylphosphatidylcholin, Cholesterol oder Cholesterylamin und gegebenenfalls übliche Zusatzstoffe, Träger oder Adjuvantien enthalten.

14. Medizinische oder diagnostische Zusammensetzungen, die mindestens eine Verbindung nach einem der Ansprüche 1 bis 12 oder eine Zusammensetzung nach Anspruch 13 enhalten.

15. Verfahren zum Einbringen von biologisch wirksamen Verbindungen, wie DNA, RNA, Ribozymen, Antisense-DNA, PNA,Peptiden, Peptoiden und Proteinen in eukariotische Zellen, wobei Verbindungen oder Zusammensetzungen nach einem der Ansprüche 1-14 mit den einzubringenden Verbindungen komplexiert und die entstandenen Komplexe mit eukariotischen Zellen in vitro in Kontakt gebracht werden.

16. Verwendung der Verbindungen oder Zusammensetzungen nach einem der Ansprüche 1-14 zur Herstellung eines Medikaments oder eines Reagenzes zum Einbringen von biologisch wirksamen Verbindungen, wie DNA, RNA, Ribozymen, Antisense-DNA, PNA, Peptiden, Peptoiden und Proteinen in eukariotische Zellen in vivo oder in vitro.

17. Verwendung nach Anspruch 16, wobei die Verbindungen in Kombination mit Enhancern verwendet werden.

## Claims

1. Compounds of the general formula **I**: 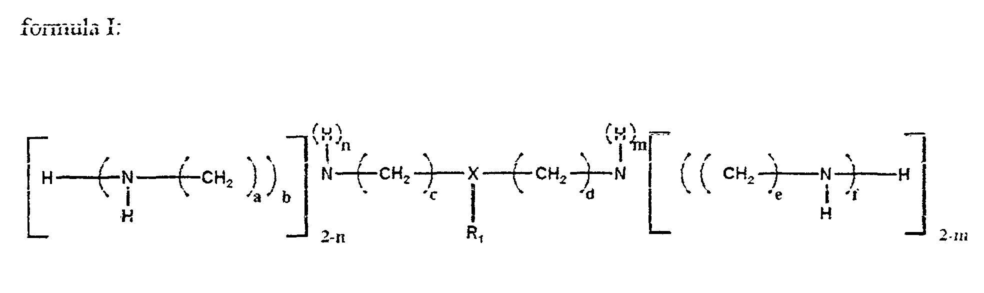 which, when a centre of asymmetry is present, occur in the D-, L- or DL-form, including sails thereof, wherein
R₁ is a lipophilic radical of the following general formula: 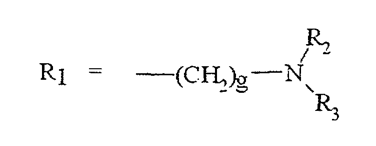 in which R₂ and R₃ are each independently of each other dodecyl, dodecenyl, tetradecyl, tetradecenyl, hexadecyl, hexadecenyl, octadecyl, octadecenyl, or other alkyl radicals, which may be saturated or unsaturated, branched or unbranched, fluorinated or non-fluorinated in all possible combinations, and are constructed from 5, 6, 7, 8, 9, 10, 11, 12, 13, 14, 15, 16, 17, 18, 19, 20, 21, 22, 23, 24, 25, 26, 27, 28, 29 or 30 carbon atoms,
and X is one of the following groupings: 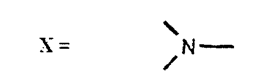 wherein m = 0 and n = 0, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, or
wherein m = 0 and n = 1, in which case g may be 1,2,3,4,5,6,7 or 8. a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6. f may be 0,1,2,3,4,5 or 6, or
wherein m = 0 and n = 2, in which case g may be 1,2,3,4,5,6,7 or 8. a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, or
wherein m = 1 and n = 1. in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, or
wherein m = 1 and n = 2, in which case g may be 1,2,3,4.5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, or
wherein m = 2 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or 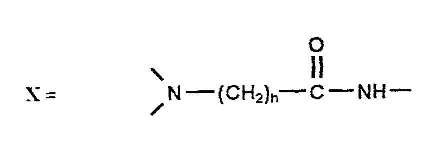 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and h may be 0,1,2,3,4,5 or 6, or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and h may be 0,1,2,3,4,5 or 6, or
wherein m = 0 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and h may be 0,1,2,3,4,5 or 6, or
wherein m = 1 and n = 1, in which case g may be 1,2,3,4,5,6,7 or 8. a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and h may be 0,1,2,3,4,5 or 6, or
wherein m = 1 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and h may be 0,1,2,3,4,5 or 6, or
wherein m = 2 and n = 2, in which case g may be 1,2,3,4,5,6,7 or 8, a may be 0,1,2,3,4,5 or 6, b may be 0,1,2,3,4,5 or 6. c may be 0,1,2,3,4,5 or 6, d may be 0,1,2,3,4,5 or 6, e may be 0,1,2,3.4,5 or 6, f may be 0,1,2,3,4,5 or 6 and h may be 0,1,2,3,4,5 or 6.
or 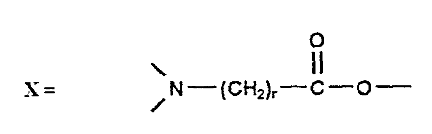 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6.
or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6,
or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6,
or
wherein m = 1 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6,
or
wherein m = 1 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6,
or
wherein m = 2 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and r may be 0,1,2,3,4,5 or 6,
or 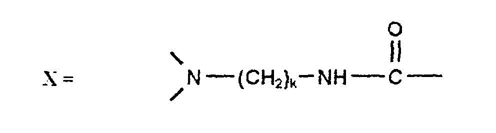 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 0 and n = 2, in which case g may be 1,2,3,4,5,6,7 or 8, a may be 0,1,2,3,4,5 or 6, b may be 0,1,2,3,4,5 or 6, c may 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 1 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 1 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 2 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6,
or 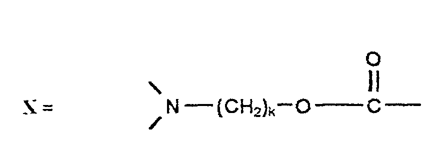 wherein m = 0 and n = 0, in which case g may be 1,2,3,4,5,6,7 or 8, a may be 0,1,2,3,4,5 or 6, b may be 0,1,2,3,4,5 or 6, c may be 0,1.2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 1 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 1 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6, or
wherein m = 2 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6, k may be 0,1,2,3,4,5 or 6,
or 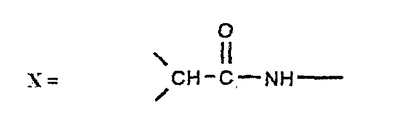 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 1, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 2, in which case g may be 1,2,3,4,5,6,7 or 8, a may be 0,1,2,3,4,5 or 6, b may be 0,1,2,3,4,5 or 6, c may be 0,1,2,3,4, or 6, d may be 0,1,2,3,4,5 or 6, e may be 0,1,2,3,4,5 or 6, f may be 0,1,2,3,4,5 of 6,
or
wherein m = 2 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or 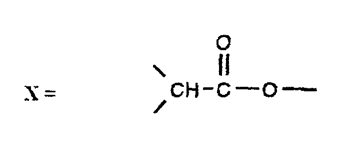 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 0 and n = 1, in which case g may be 1,2,3,4,5,6,7 or 8, a may be 0,1,2,3,4,5 or 6. b may be 0,1.2,3,4,5 or 6, c may be 0,1,2,3,4,5 or 6, d may be 0,1.7,3,4,5 or 6, e may be 0,1,2,3,4,5 or 6, f may.. be 0,1,2,3,4,5 or 6,
or
wherein m = 0 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 1, in which case g may be 1,2,3,4,5,6,7 or 8, a may be 0,1,2,3,4, or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 2, in which case g may be 1,2,3,4,5,6,7 or 8, a may be 0,1.2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 2 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or 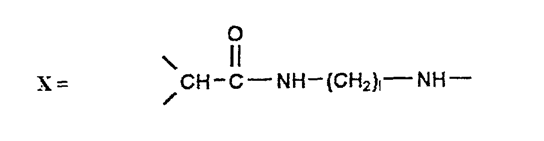 wherein m = 0 and n = 0, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and l may be 0,1,2,3,4,5 or 6, or
wherein m = 0 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4, 5 or 6, e may be 0,1,2,3,4,5 or 6, f may be 0,1,2,3,4,5 or 6 and 1 may be 0,1,2,3,4,5 or 6, or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and 1 may be 0,1,2,3,4,5 or 6, or
wherein m = 1 and n = 1, in which case g may be 1,2,3,4,5,6,7 or 8, a may be 0,1,2,3,4,5 or 6. b may be 0,1,2,3,4,5 or 6, c may be 0,1,2,3,4,5 or 6, d mar be 0,1,2,3,4, or 6, e may be 0,1,2,3,4,5 or 6, f may be 0,1,2,3,4,5 or 6 and I may be 0,1,2,3,4,5 or 6, or
wherein m = 1 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and I may be 0,1,2,3,4,5 or 6, or
wherein m = 2 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6 and 1 may be 0,1,2,3,4,5 or 6,
or 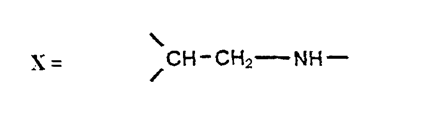 wherein m = 0 and n = 0, in which case g may be 1,2,3,4 5,6,7 or 8, a may be 0,1,2,3,4,5 or 6. b may be 0,1,2,3,4,5 or 6, c may be 0,1,2,3,4, 5 or 6, d may be 0,1,2,3,4,5 or 6, e may be 0,1,2,3,4,5 or 6, f may be 0,1,2,3,4,5 or 6,
or
wherein m = 0 and n = 1, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 1, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 2 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or 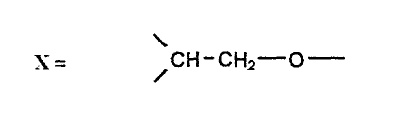 wherein m = 0 and n = 0, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 0 and n = 1, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6. e may be 0,1,2,3,4,5 or 6, f may be 0,1,2,3,4,5 or 6,
or
wherein m = 0 and n = 2, in which case g may be 1,2,3,4,5,6,7 or 8, a may be 0,1,2,3,4,5 or 6, b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 1, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 1 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
or
wherein m = 2 and n = 2, in which case g may be 1,2,3,4,5,6,7 or 8, a may be 0,1,2,3,4,5 or 6. b may be 0,1,2,3,4,5 or 6, c may be 0,1,2,3,4,5 or 6, d may be 0,1,2,3,4,5 or 6, e may be 0,1,2,3,4,5 or 6, f may be 0,1,2,3,4,5 or 6,
with the proviso that in all of the above cases b is less than or equal to 1 when a = 0 and f is less than or equal to 1 when e = 0, and the case is excluded that a = b = c = d = e = f = 0.

2. Compounds and salts thereof according to claim 1 having the following structures: 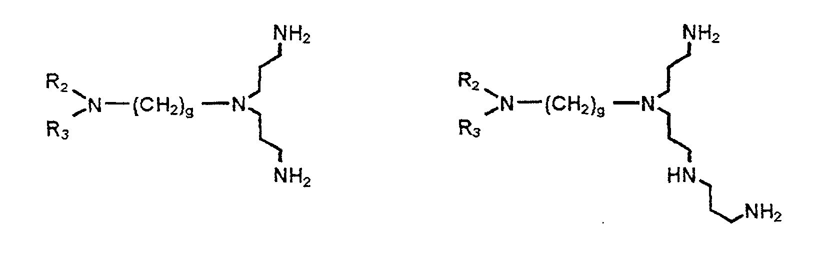 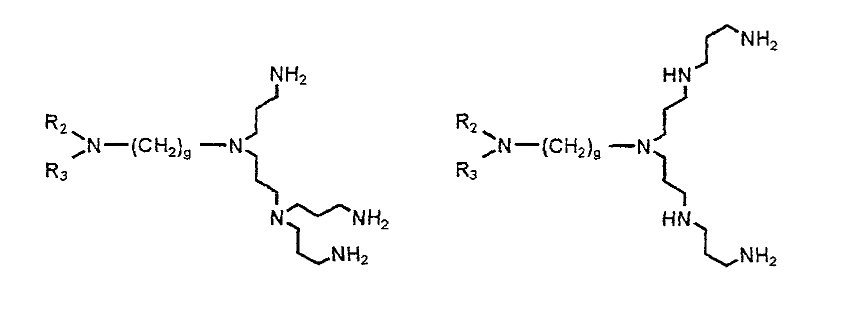 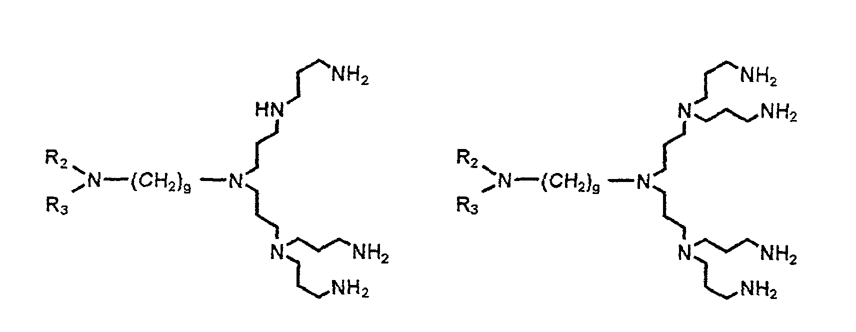 wherein g may be 1,2,3,4,5,6,7 or 8 and R₂ and R₃ may each, independently of each other, be dodecyl, dodecenyl, tetradecyl, tetradecenyl, hexadecyl, hexadecenyl, octadecyl or octadecenyl.

3. Compounds and salts thereof according to claim 1 having the following structures: 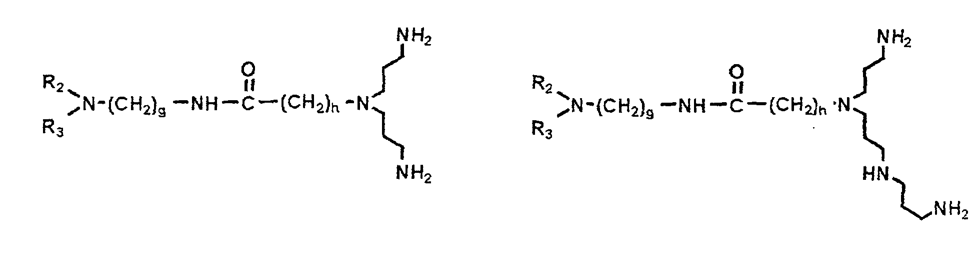 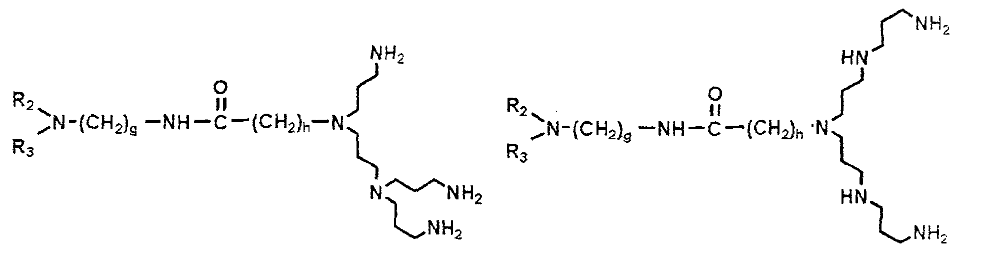 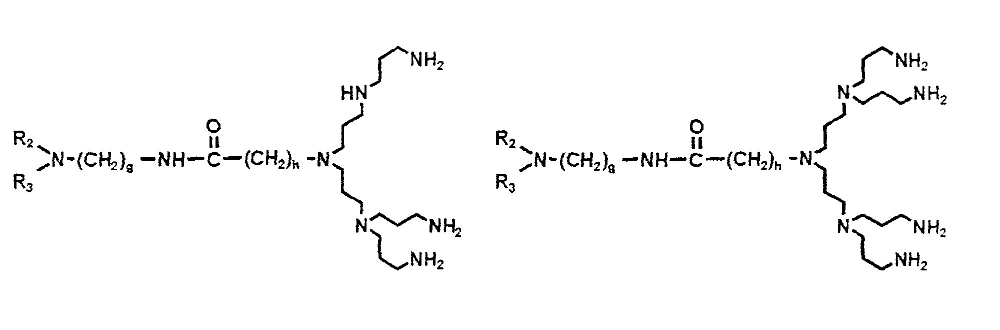 wherein h may be 0,1,2,3,4,5 or 6 and g may be 1,2,3,4,5,6,7 or 8 and R₂ and R₃ may each. independently of each other, be dodecyl, dodecenyl, tetradecyl, tetradecenyl, hexadecyl, hexadecenyl, octadecyl or octadecenyl.

4. Compounds and salts thereof according to claim 1 having the following structures: 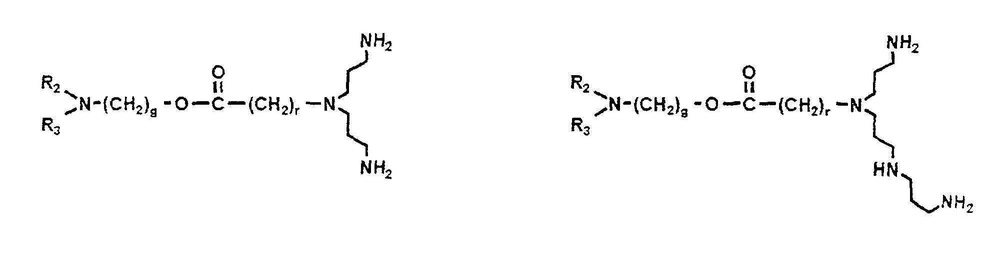 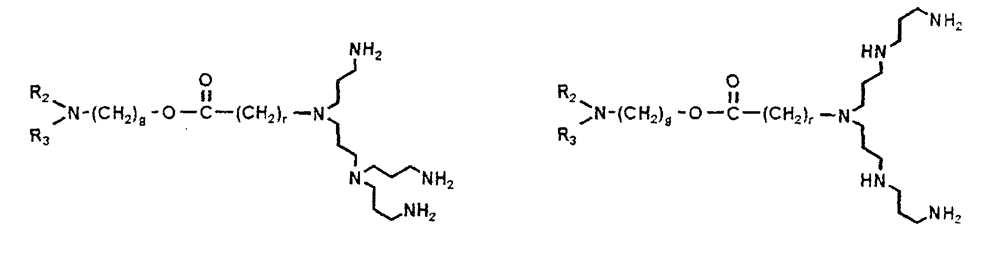 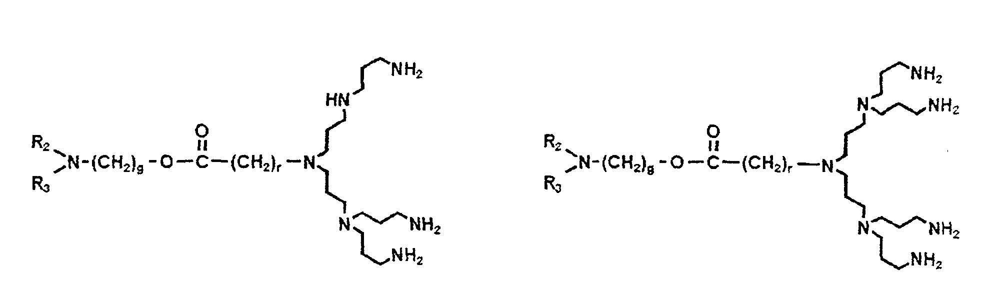 wherein r may be 0,1,2,3,4,5 or 6 and g may be 1,2,3,4,5,6,7 or 8 and R₂ and R₃ may each, independently of each other, be dodecyl, dodecenyl, tetradecyl, tetradecenyl, hexadecyl, hexadecenyl, octadecyl or octadecenyl.

5. Compounds and salts thereof according to claim 1 having the following structures: 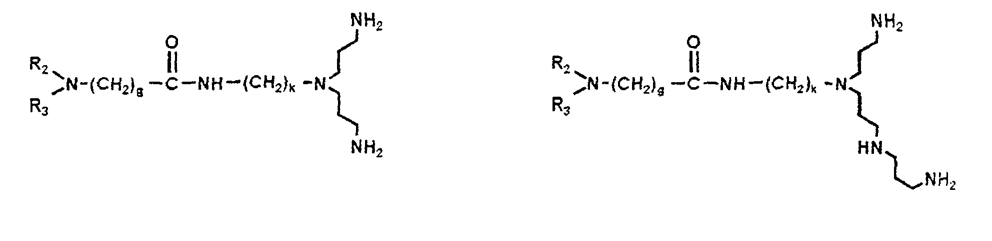 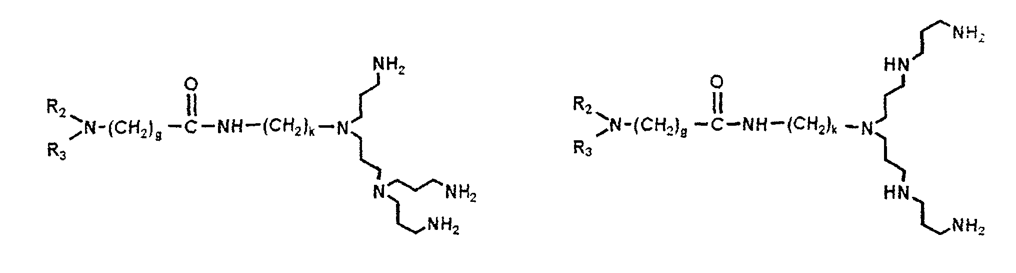 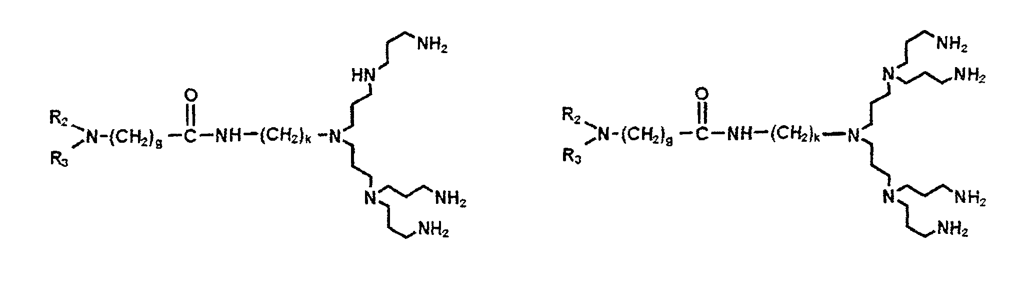 wherein k may be 1,2,3,4,5 or 6 and g may be 1,2,3,4,5,6,7 or 8 and R₂ and R₃ may each, independently of each other, be dadecyl, dodecenyl, tetradecyl, tetradecenyl, hexadecyl, hexadecenyl, octadecyl or octadecenyl.

6. Compounds and salts thereof according to claim 1 having the following structures: 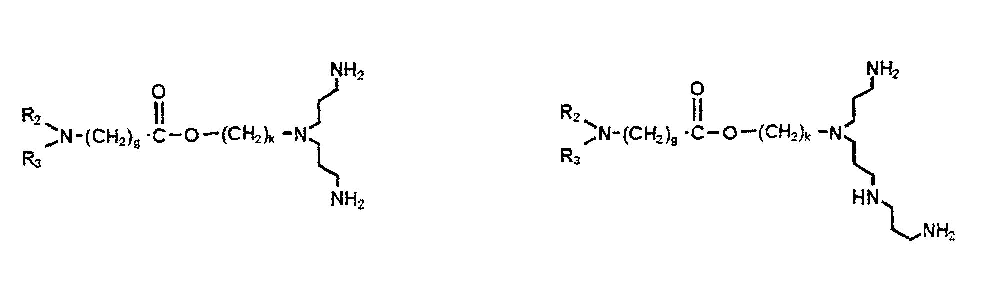 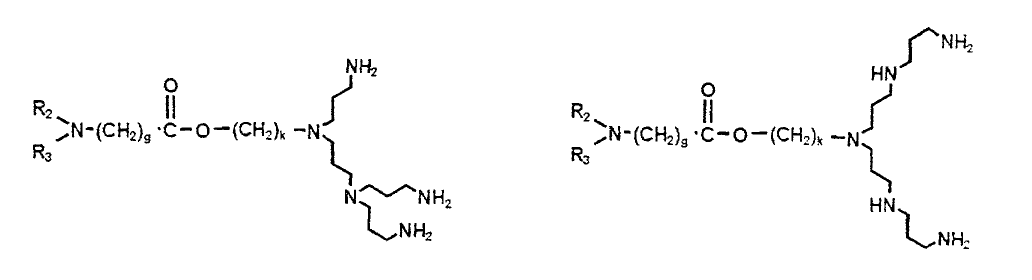 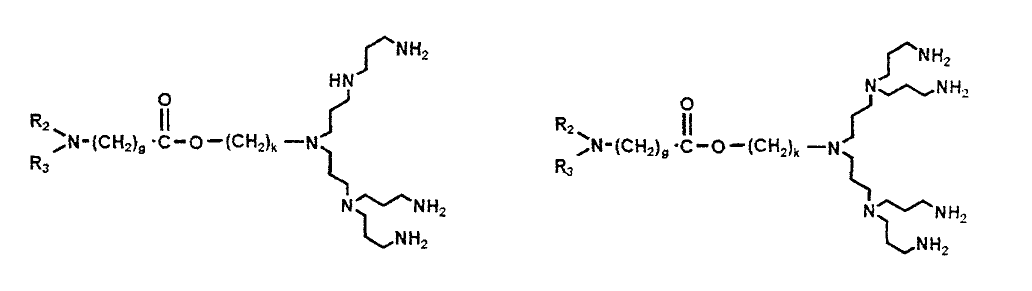 wherein k may be 1,2,3,4,5 or 6 and g may be 1,2,3,4,5,6,7 or 8 and R₂ and R₃ may each, independently of each other, be dodecyl, dodecenyl, tetradecyl, tetradecenyl, hexadecyl, hexadecenyl, octadecyl or octadecenyl.

7. Compounds and salts thereof according to claim 1 having the following structures: 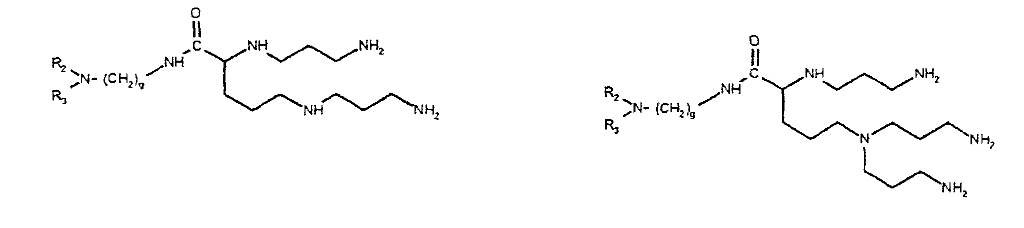 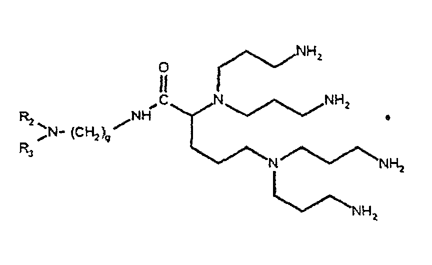 wherein g may be 1,2,3,4, 5,6,7 or 8 and R₂ and R₃ may each independently of each other, be dodecyl, dodecenyl, tetradecyl, tetradecenyl, hexadecyl, hexadecenyl, octadecyl or octadecenyl.

8. Compounds and salts thereof according to claim 1 having the following structures: 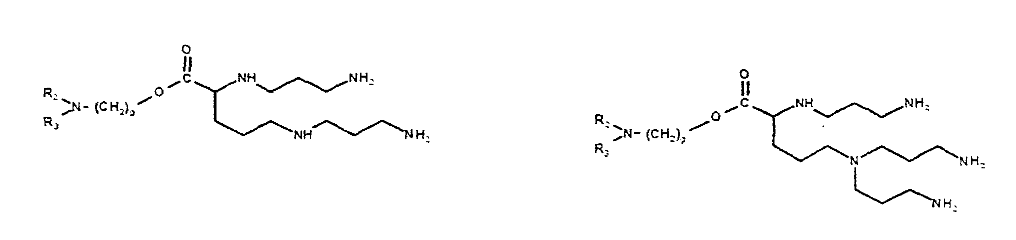 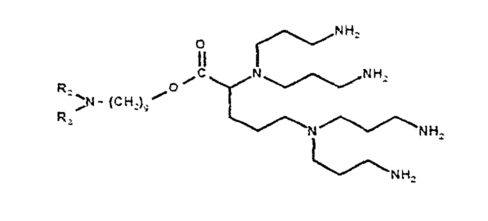 wherein g may be 1,2,3,4, 5,6,7 or 8 and R₂ and R₃ may each, independently of each other, be dodecyl, dodecenyl, tetradecyl, tetradecenyl, hexadecyl, hexadecenyl, octadecyl or octadecenyl.

9. Compounds and salts thereof according to claim I having the following structures: 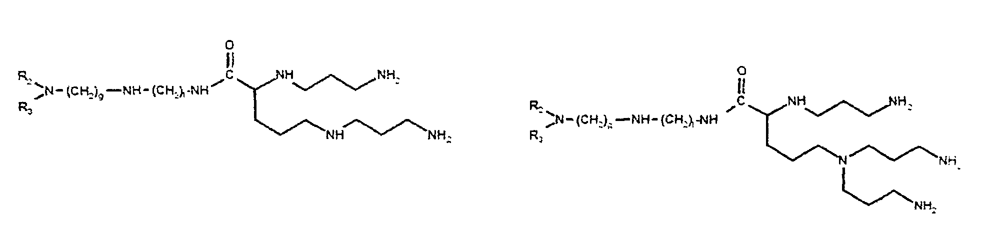 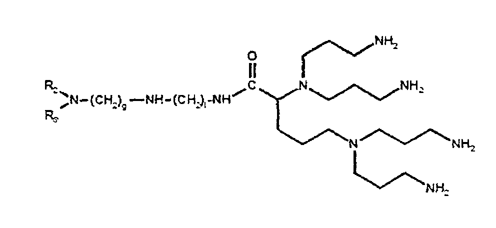 wherein 1 may be 0,1,2,3,4,5 or 6 and g may be 1,2,3,4, 5,6,7 or 8 and R₂ and R₃ may each, independently of each other, be dodecyl, dodecenyl, tetradecyl, tetradecenyl, hexadecyl, hexadecenyl, octadecyl or octadecenyl.

10. Compounds and salts thereof according to claim 1 having the following structures: 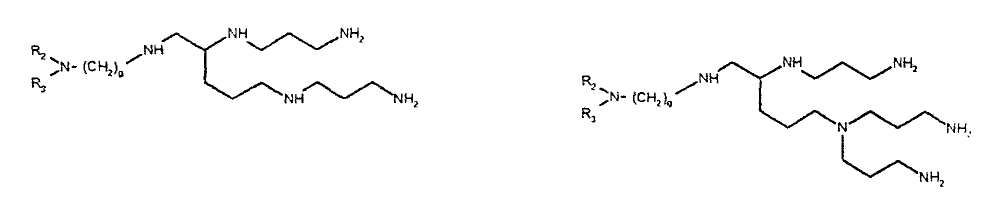 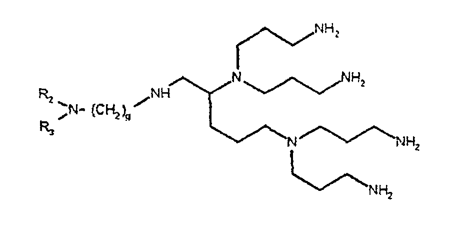 wherein g may be 1,2,3,4, 5,6,7 or 8 and R₂ and R₃ may each, independently of each other, be dodecyL dodecenyl, tetradecyl, tetradecenyl, hexadecyl, hexadecenyl, octadecyl or octadecenyl.

11. Compounds and salts thereof according to claim 1 having the following structures: 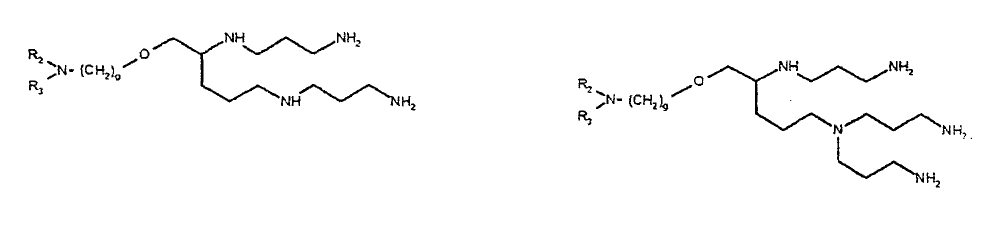 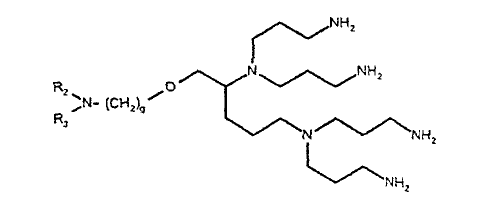 wherein g may be 1,2,3,4, *5,6,7* or 8 and R₂ and R₃ may each, independently of each other, be dodecyl, dodecenyl, tetradecyl, tetradecenyl, hexadecyl, hexadecenyl, octadecyl or octadecenyl.

12. Compounds and salts thereof according to claim 1 having the following structures:

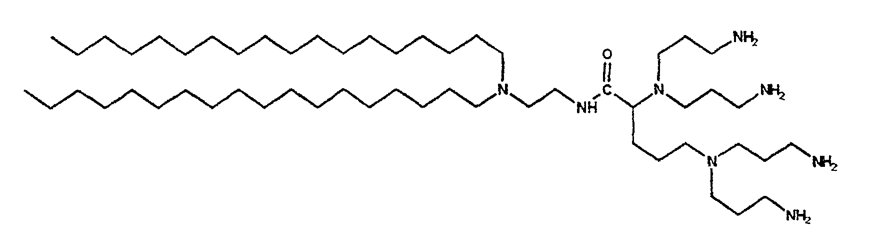

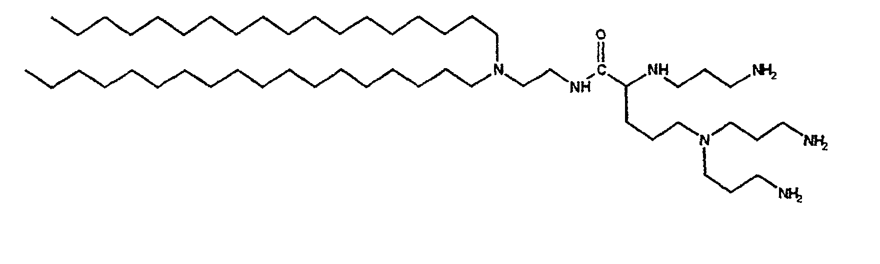

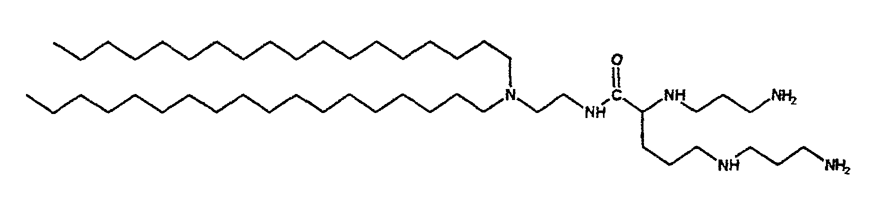

13. Compositions that comprise at least one of the compounds of claims 1 to 12 with or without colipids, such as, for example, dioleoyl phosphatidyl ethanolaimne (DOPE), dioleoyl phosphatidyl choline, cholesterol or cholesteryl amine and, optionally, customary additives, carriers or adjuvants.

14. Medicinal or diagnostic compositions that comprise at least one compound according to any one of claims 1 to 12 or a composition according to claim 13.

15. A method of introducing biologically active compounds, such as DNA, RNA, ribozymes. antisense DNA, PNA, peptides, peptoids and proteins, into eukaryotic cells, wherein compounds or compositions according to any one of claims 1 to 14 are complexed with the compounds to be introduced, and the resulting complexes are brought into contact *in vitro* with eukaryotic cells.

16. The use of the compounds or compositions according to any one of claims 1 to 14 in the preparation of a medicament or a reagent for the *in vivo* or *in vitro* introduction of biologically active compounds, such as DNA, RNA, ribozymes, antisense DNA, PNA, peptides, peptoids and proteins, into eukaryotic cells.

17. Use according to claim 16, wherein the compounds are used in combination with enhancers.

## Revendications

1. Composés de formule générale I : 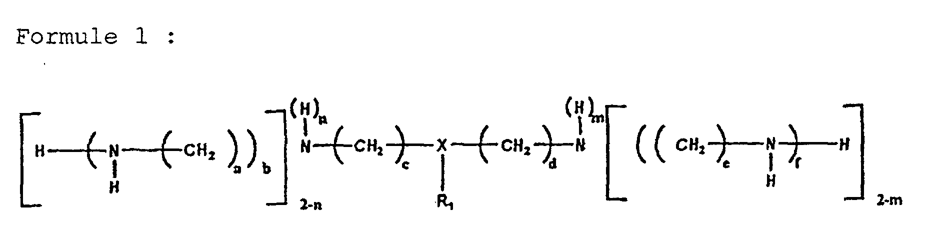 qui existent sous la forme D, L ou DL lorsqu'un centre d'asymétrie existe, y compris leurs sels, dans lesquels
R₁ représente un résidu lipophile de la formule générale suivante : 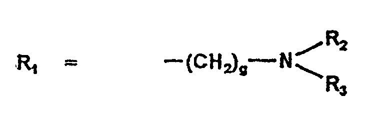 dans laquelle
R₂ et R₃ représentent indépendamment l'un de l'autre un groupe dodécyle, dodécényle, tétradécyle, tétradécényle, hexadécyle, hexadécényle, octadécyle, octadécényle, ou d'autres résidus alkyle qui peuvent être saturés ou insaturés, ramifiés ou non ramifiés, fluorés ou non fluorés et sont constitués de 5, 6, 7, 8, 9, 10, 11, 12, 13, 14, 15, 16, 17, 18, 19, 20, 21, 22, 23, 24, 25, 26, 27, 28, 29 ou 30 atomes de carbone,
et X représente un des groupements suivants : 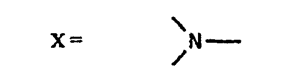 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou 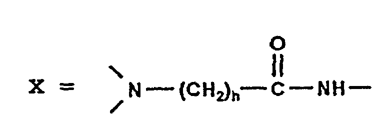 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et h = 0, 1, 2, 3, 4, 5 ou 6, ou 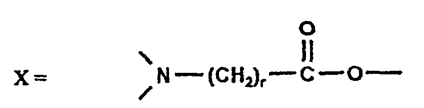 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et r = 0, 1, 2, 3, 4, 5 ou 6,
ou 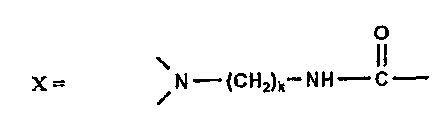 dans lesquels m = 0 et n = 0 et on peut avoir pour ce *cas* g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6,
ou 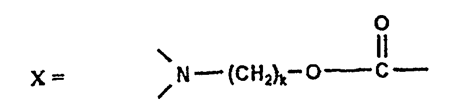 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6, ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, k = 0, 1, 2, 3, 4, 5 ou 6,
ou 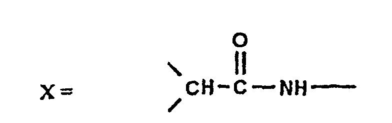 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 1 et on peut avoir pour ce cas g - 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou 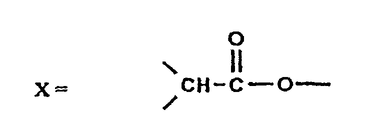 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou 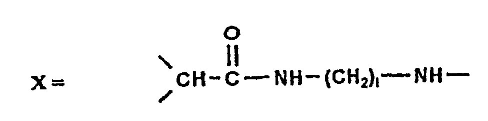 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6, ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6, ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6, ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6, ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6, ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, et 1 = 0, 1, 2, 3, 4, 5 ou 6,
ou 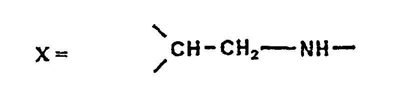 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 2 et on peut avoir pour ce *cas* g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou 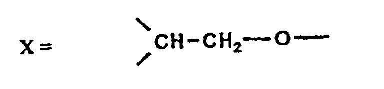 dans lesquels m = 0 et n = 0 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 0 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 1 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 1 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6,
ou
dans lesquels m = 2 et n = 2 et on peut avoir pour ce cas g = 1, 2, 3, 4, 5, 6, 7 ou 8, a = 0, 1, 2, 3, 4, 5 ou 6, b = 0, 1, 2, 3, 4, 5 ou 6, c = 0, 1, 2, 3, 4, 5 ou 6, d = 0, 1, 2, 3, 4, 5 ou 6, e = 0, 1, 2, 3, 4, 5 ou 6, f = 0, 1, 2, 3, 4, 5 ou 6, à la condition que dans tous les cas précédents, b soit égal ou inférieur à 1, lorsque a = 0 et f soit égal ou inférieur à 1, lorsque e = 0, et le cas où a = b = c = d = e = f = 0 soit exclu.

2. Composés et leurs sels selon la revendication 1 avec les structures suivantes : 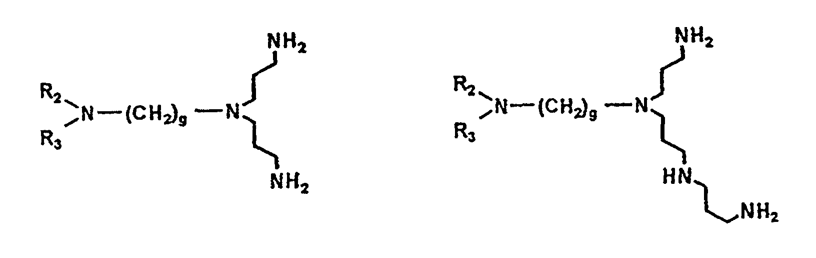 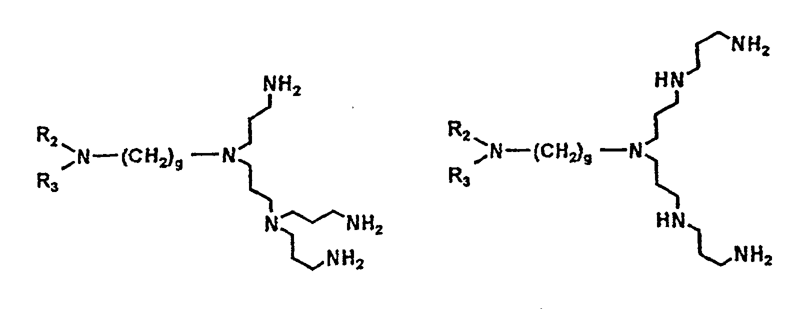 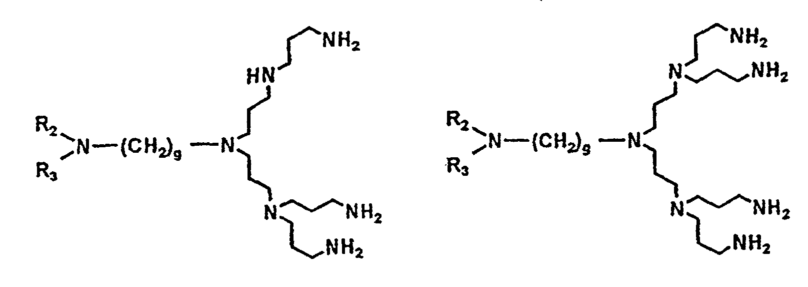 dans lesquelles g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

3. Composés et leurs sels selon la revendication 1 avec les structures suivantes : 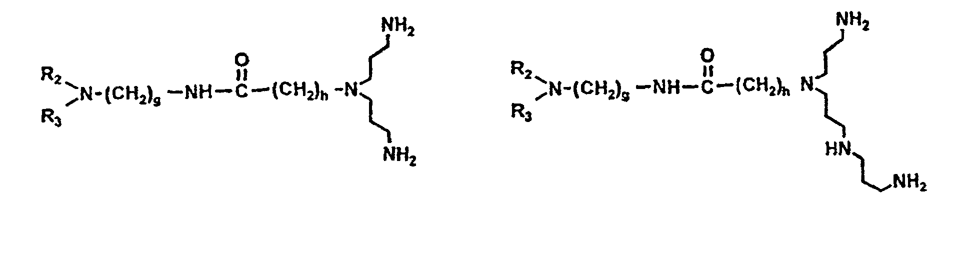 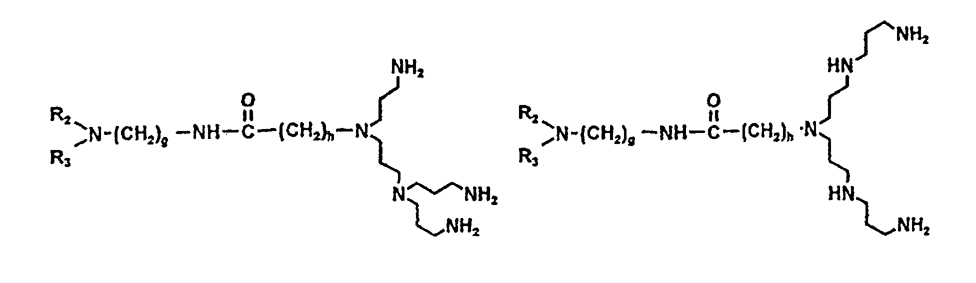 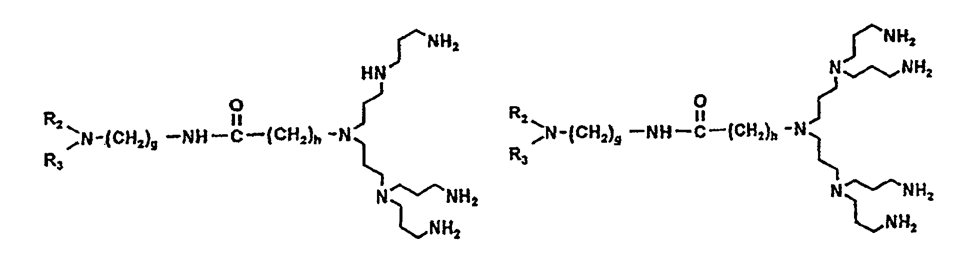 dans lesquelles h = 1, 2, 3, 4, 5 ou 6 et g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

4. Composés et leurs sels selon la revendication 1 avec les structures suivantes : 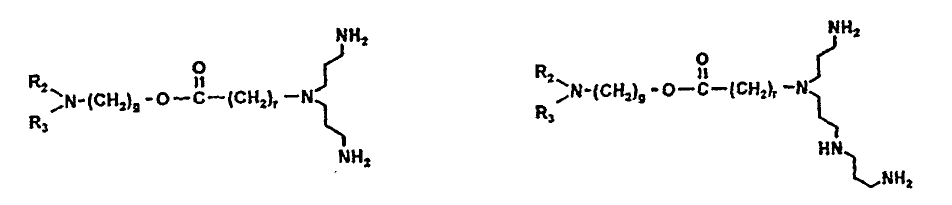 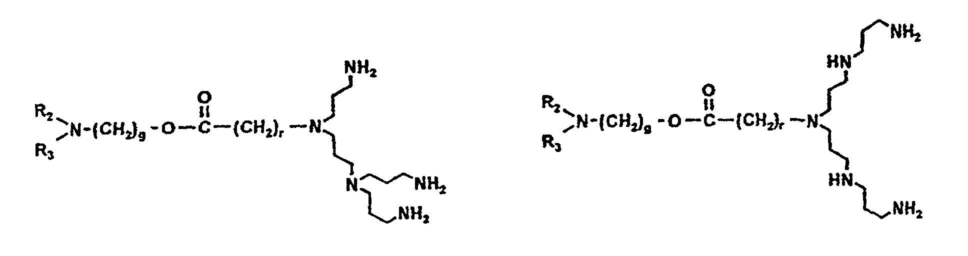 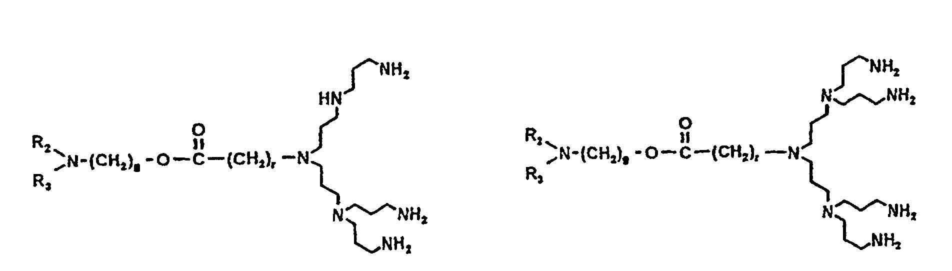 dans lesquelles r = 1, 2, 3, 4, 5 ou 6 et g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

5. Composés et leurs sels selon la revendication 1 avec les structures suivantes : 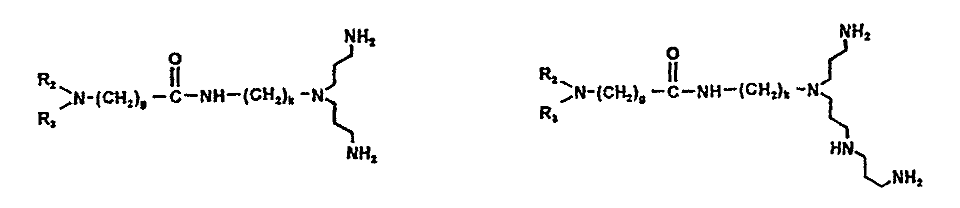 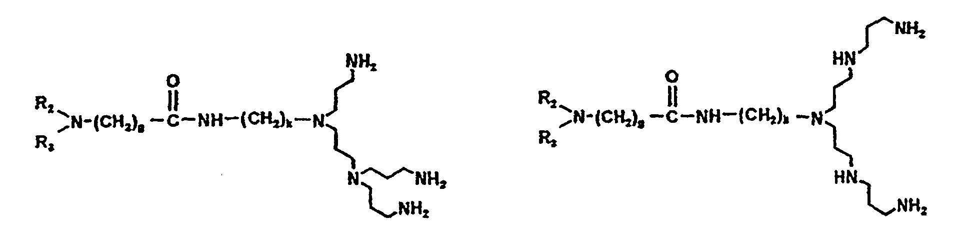 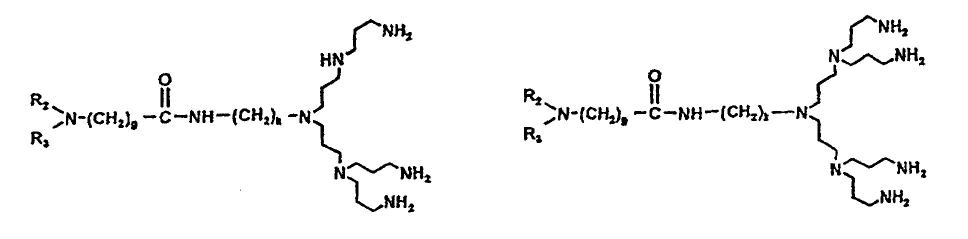 dans lesquelles k = 1, 2, 3, 4, 5 ou 6 et g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

6. Composés et leurs sels selon la revendication 1 avec les structures suivantes : 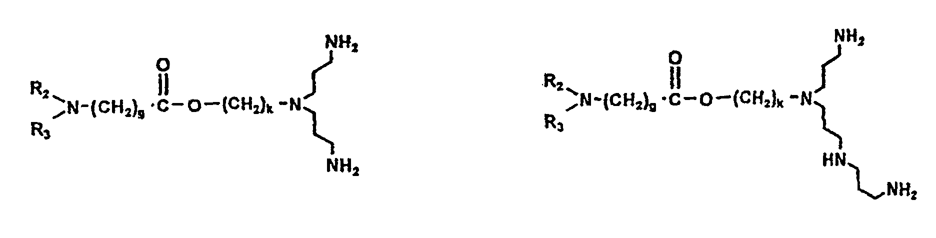 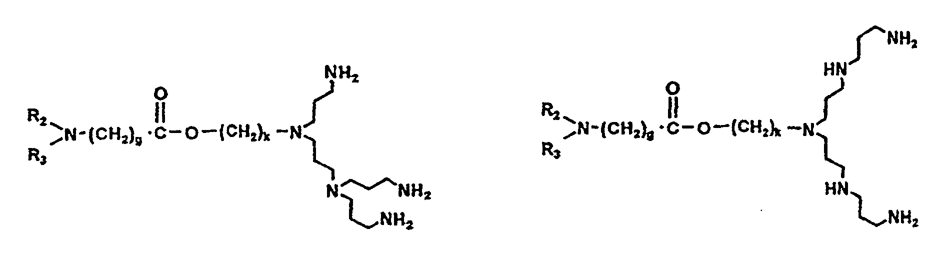 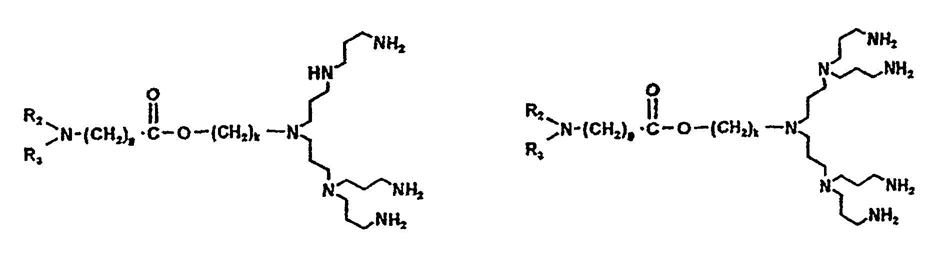 dans lesquelles k = 1, 2, 3, 4, 5 ou 6 et g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

7. Composés et leurs sels selon la revendication 1 avec les structures suivantes : 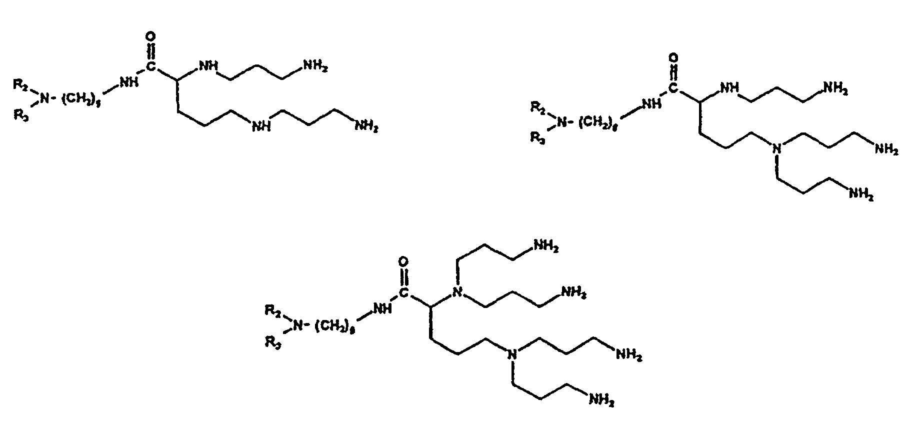 dans lesquelles g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

8. Composés et leurs sels selon la revendication 1 avec les structures suivantes : 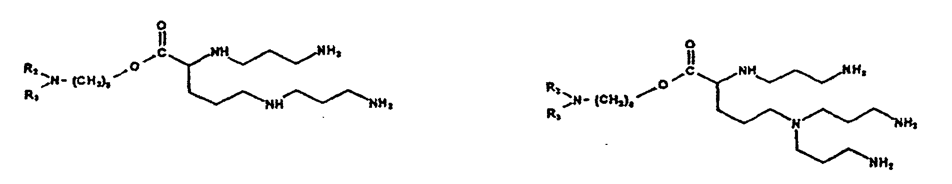 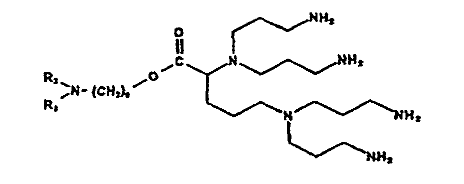 dans lesquelles g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

9. Composés et leurs sels selon la revendication 1 avec les structures suivantes : 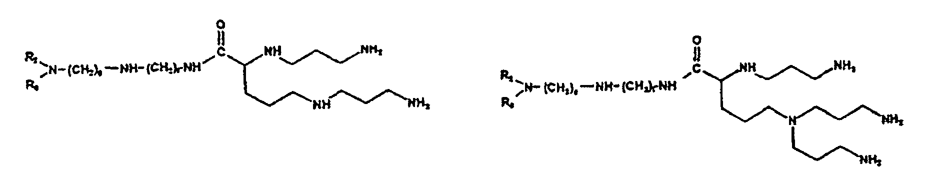 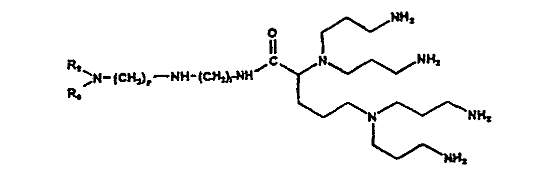 dans lesquelles 1 = 1, 2, 3, 4, 5 ou 6 et g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

10. Composés et leurs sels selon la revendication 1 avec les structures suivantes : 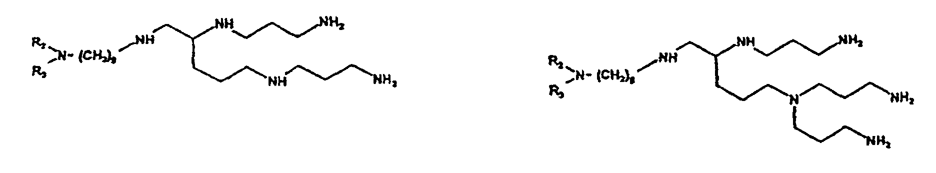 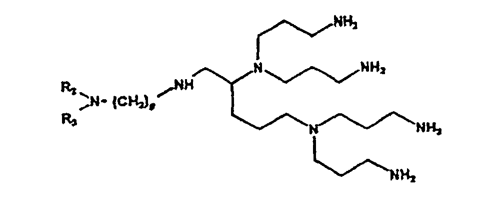 dans lesquelles g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

11. Composés et leurs sels selon la revendication 1 avec les structures suivantes : 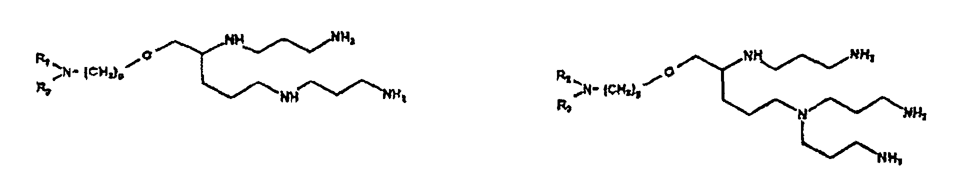 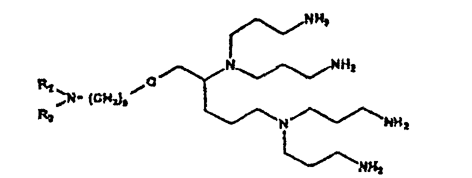 dans lesquelles g = 1, 2, 3, 4, 5, 6, 7 ou 8 et R₂ et R₃ peuvent représenter indépendamment l'un de l'autre un groupe dodécyle, dodécényle, tétradécyle, tétradécényle, hexadécyle, hexadécényle, octadécyle ou octadécényle.

12. Composés et leurs sels selon la revendication 1 avec les structures suivantes :

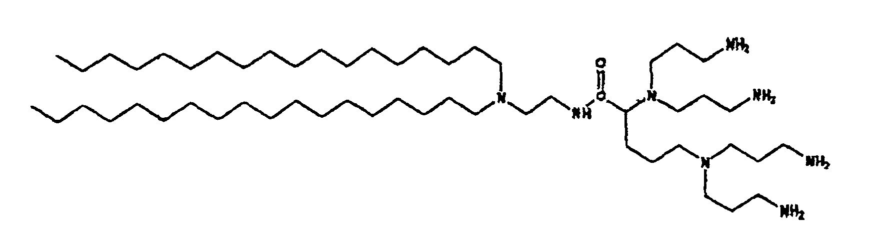

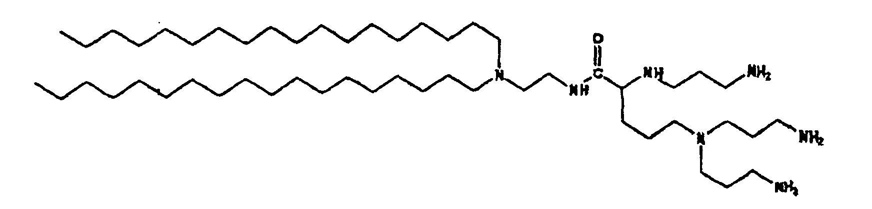

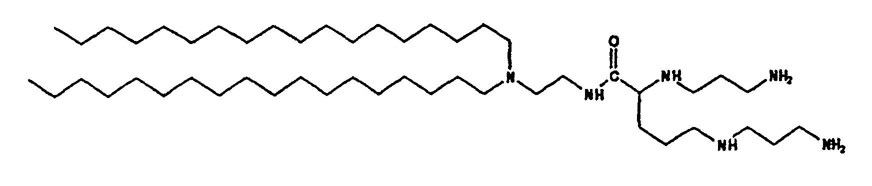

13. Compositions qui contiennent au moins un des composés selon les revendications 1 à 12 avec ou sans colipides, comme par exemple la dioléoylphosphatidyléthanolamine (DOPE), la dioléoylphosphatidylcholine, le cholestérol ou la cholestérylamine et éventuellement des additifs, véhicules ou adjuvants.

14. Compositions de médicament ou pour le diagnostic qui contiennent au moins un des composés selon l'une quelconque des revendications 1 à 12 ou une composition selon la revendication 13.

15. Procédé pour introduire des composés biologiquement efficaces, comme l'ADN, l'ARN, les ribozymes, l'ADN antisens, les acides nucléiques peptidiques (PNA), les peptides, les peptoïdes et les protéines dans des cellules eucaryotes, dans lequel des composés ou compositions selon l'une quelconque des revendications 1 à 14 sont complexés avec les composés à introduire et les complexes formés sont amenés en contact avec des cellules eucaryotes *in vitro.*

16. Utilisation des composés ou compositions selon l'une quelconque des revendications 1 à 14 pour préparer un médicament ou un réactif pour introduire des composés biologiquement efficaces, comme l'ADN, l'ARN, les ribozymes, l'ADN antisens, les acides nucléiques peptidiques (PNA), les peptides, les peptoïdes et les protéines dans des cellules eucaryotes in vivo ou in vitro.

17. Utilisation selon la revendication 16, dans laquelle les composés sont utilisés en combinaison avec des exhausteurs.
